# EUROPEAN PATENT SPECIFICATION

(11) **EP 2 691 077 B1**
(45) Date of publication and mention of the grant of the patent: **09.05.2018**
(21) Application number: 12717447.2
(22) Date of filing: 26.03.2012
(51) Int. Cl.: A61K 9/00, A61K 47/14, A61K 47/26, A61K 31/34, A61K 31/60, A61P 17/10

(54) **SALICYLIC ACID TOPICAL FORMULATION**
TOPISCHE FORMULIERUNG ENTHALTEND SALICYLSÄURE
PREPARATION TOPIQUE CONTENANT DE L'ACIDE SALICYLIQUE

(30) Priority: 31.03.2011 GB 201105410; 29.06.2011 GB 201111013
(43) Date of publication of application: 05.02.2014
(73) Proprietor: Integumen Ireland Limited, Dublin (IE)
(72) Inventor: ABBOTT, Steven John, Wetherby, Yorkshire LS22 7TW (GB); COVE, Jonathan Howard, Wetherby, Yorkshire LS22 7TW (GB); DONOGHUE, Gavin, Wetherby, Yorkshire LS22 7TW (GB); GREGORY, Sarah-Jayne, Wetherby, Yorkshire LS22 7TW (GB)
(74) Representative: Glansdorp, Freija Gwendolyn
(86) International application number: PCT/GB2012/050665
(87) International publication number: WO 2012/131348

(56) References cited:
- WO-A1-01/01949
- WO-A2-02/096435
- GB-A- 2 283 421
- US-A1- 2003 194 385
- US-A1- 2007 292 355
- US-A1- 2008 194 518

## Description

### Field of the invention

This claimed invention relates to topical skin treatment formulations containing salicylic acid and to the preparation and use of such formulations.

### Background to the invention

Salicylic acid (2-hydroxybenzoic acid) is a known anti-acne agent. It is a keratolytic which is believed to loosen the binding between stratum corneum cells, when topically applied to the skin, causing the outer layers of the skin to shed away from the pores. It is therefore widely used to unblock pores to help prevent whiteheads and blackheads becoming inflamed (Waller JM, Dreher F, Behnam S, Ford C, Lee C, Tiet T, Weinstein GD, Maibach HI, "'Keratolytic' properties of benzoyl peroxide and retinoic acid resemble salicylic acid in man", Skin Pharmacol Physiol 2006;19: 283-9; Eady et al, 1996, J Dermatol Treat, 7: 93-96; Cunliffe WJ et al, 2004, Clin Dermatol, 22(5): 367-74).

The keratolytic action of salicylic acid can be concentration dependent and in cases it is necessary to combine it with other actives to increase efficacy, especially against inflammatory lesions (see for example Touitou E, Godin B, Shumilov M, Bishouty N, Ainbinder D, Shouval R, Ingber A, Leibovici V, "Efficacy and tolerability of clindamycin phosphate and salicylic acid gel in the treatment of mild to moderate acne vulgaris", J Eur Acad Dermatol Venereol 2008; 22: 629-31; and GB-2 018 589 which describes the use of salicylic acid together with the well-known anti-acne active benzoyl peroxide). There is therefore a need to find alternative anti-acne formulations in which the efficacy of salicylic acid can be boosted by delivering it more efficiently to the desired areas of the skin.

However, salicylic acid is also a potential irritant, particularly at higher concentrations: according to the Cosmetic Ingredient Review published in *International Journal of Toxicology,* 22(Suppl. 3): 1-108, 2003, the acid and its salts need to be "formulated to avoid skin irritation". Thus, it may be undesirable to try to increase the effectiveness of a topical salicylic acid-containing formulation simply by increasing the concentration of the acid.

US2007/0292355 discloses a composition comprising salicylic acid (see examples 13a) part B and example 13 b) page 34) in a solvent system from which DMI, a glyceryl fatty acid ester (Glyceryl Monostearate/PEG) and an alcohol. The preparation is for topical application and could comprise also usnic acid. Another factor which can affect the efficacy of salicylic acid formulations, when applied in vivo, is the tendency of the molecule to pass rapidly through the stratum corneum. This can limit the duration, and often the magnitude, of its therapeutic effects.

It is an aim of the present invention to provide novel topical skin treatment formulations containing salicylic acid or derivatives thereof, which formulations can deliver the salicylic acid or derivative in an efficient and targeted manner, both to the stratum corneum and ultimately to the pilosebaceous follicles, and which can ideally provide longer lasting therapeutic activity.

### Statements of the invention

According to the present invention there is provided a topical skin treatment formulation containing salicylic acid or a derivative thereof dissolved in a solvent system, wherein the solvent system comprises:
(i) dimethyl isosorbide (DMI);
(ii) a C1 to C9 alkyl salicylate; and
(iii) a glyceryl fatty acid ester.

This specific combination of solvents has been found capable of delivering salicylic acid to the stratum corneum and pilosebaceous follicles following topical application to the skin surface, with a surprisingly high speed and depth of penetration, as illustrated by the examples below. This in turn can increase the speed of onset of the keratolytic action, and the depth at which the action occurs. The invented solvent combination has also surprisingly been found capable of improving the persistence of the salicylic acid in the stratum corneum following its initial application. The invented formulation is thus expected to be of value as a keratolytic and/or anti-acne agent.

According to the present invention, it is necessary to use a specific mixture of solvents which together dissolve the salicylic acid or derivative, and deliver it effectively through the skin following topical application, and which are also acceptable for pharmaceutical and/or cosmetic use. It is believed, although we do not wish to be bound by this theory, that a formulation according to the invention can allow the salicylic acid or derivative to diffuse efficiently through the layers of the stratum corneum, and thence, ultimately, to the pilosebaceous follicles where it can act to loosen microcomedones and hence to reduce follicle blockages.

In an embodiment of the invention, the solvent system comprises, in addition to the components (i) to (iii):
(iv) an alcohol.

In an embodiment, the solvent system comprises, in addition to the components (i) to (iii) and optionally (iv):
(v) a polyoxyalkylene-based solvent selected from polyoxyalkylene glyceryl esters, polyalkylene glycols and mixtures thereof.

In an embodiment, the solvent system comprises, in addition to the components (i) to (iii) and optionally (iv) and/or (v):
(vi) 2-pyrrolidone or a C1 to C12 alkyl pyrrolidone.

In an embodiment, the solvent system comprises, in addition to the components (i) to (iii) and optionally (iv), (v) and/or (vi):
(vii) a C1 to C4 alkyl glucose ester.

In an embodiment, the solvent system comprises, in addition to the components (i) to (iii) and optionally (iv), (v), (vi) and/or (vii):
(viii) a C1 to C4 alkyl pyruvate.

In some embodiments of the invention, however, it may be preferred for the solvent system not to comprise the pyrrolidone component (vi) and/or the pyruvate component (viii).

The solvents (i) to (iii), optionally together with (iv), (v), (vi), (vii) and/or (viii), can together help to keep the active substance in a soluble form so that it can continue to diffuse through the skin over a longer period following topical application of the formulation - for example for 4 hours or more, or for 8 or 12 or even 16 or 20 hours or more. They also appear to lend substantivity to the formulation, as demonstrated in the examples below, causing it to linger on the skin - and thus to continue to exert its anti-acne effects - after application. This enhanced persistence in turn allows the formulation to be applied for example twice or even once daily, yet to provide a sustained beneficial effect for an extended period after each application. In an embodiment, therefore, the formulation of the invention is a sustained release and/or a sustained action formulation. In the present invention, the solvent system consists essentially of components (i) to (iii), or of components (i) to (iii) together with one or more of the optional components (iv) to (viii). In an embodiment, the solvent system comprises, or consists essentially of, components (i) to (v), in the absence of components (vi) to (viii). In an embodiment it comprises, or consists essentially of, components (i) to (iv), in the absence of components (v) to (viii). In an embodiment it comprises, or consists essentially of, components (i) to (iii) together with either or both of components (iv) and (v), and optionally also with component (vii). In an embodiment it comprises, or consists essentially of, components (i) to (iii) together with either or both (suitably both) of (A) component (v) and (B) ethanol and/or tetrahydrofurfuryl alcohol (THFA). The salicylic acid or derivative is present in the formulation as an active substance. It is typically present as an anti-acne agent, and/or as a keratolytic agent. It may be present for its proteolytic activity, to loosen the structure of the stratum corneum, to unblock pilosebaceous follicles, and/or to reduce numbers of microcomedones.

A salicylic acid "derivative" may be a salt, for example a metal salt or ammonium salt or vitamin salt. Suitable metal salts include the alkali metal salts (for example the sodium and potassium salts, in particular the former) and the alkaline earth metal salts (for example the calcium and magnesium salts, in particular the former). A metal salicylate may also be selected from bismuth salicylate, bismuth subsalicylate and transition metal salts such as zinc, copper or titanium salts. Other salicylate salts include MEA-salicylate and TEA-salicylate. Other salicylic acid derivatives include salicylic acid esters, in particular alkyl esters (of which the alkyl group may be either straight chain or branched, or may incorporate one or more cyclic groups), more particularly C1-C20 or C12-C15 or C1-C10 or C1-C6 alkyl esters such as in particular methyl salicylate ("wintergreen"), capryloyl salicylic acid and homosalate (3,3,5-trimethylcyclohexyl 2-hydroxybenzoate). Further derivatives include benzyl salicylate and betaine salicylate. In cases they may include substituted salicylic acids and salts thereof, such as 4-aminosalicylic acid, thiosalicylic acid and their salts and esters.

A salicylic acid derivative may be selected from 4-aminosalicylic acid and its salts and esters; capryloyl salicylic acid; glucosamine salicylate; MEA-salicylate; TEA-salicylate; metal salicylates such as those listed above; thiosalicylic acid; benzyl salicylate; amyl (pentyl) and isoamyl (isopentyl) salicylates; azeloyl diethyl salicylate; betaine salicylate; butyloctyl salicylate; chitosan salicylate; dipropylene glycol and glycol salicylates; ethylhexyl (octyl) salicylate; hexanediol disalicylate; hexyl dodecylsalicylate; hexyl salicylate; isocetyl salicylate; isodecyl salicylate; menthyl salicylate; methyl salicylate; myristyl salicylate; niacinamide salicylate; phenyl salicylate; potassium methoxysalicylate; pyridoxine salicylate; silanediol salicylate; tridecyl salicylate; trimethylsilyl trimethylsiloxy salicylate; zinc thiosalicylate; 4-acetaminophenyl salicylate; cyclohexanol, 3,3,5- trimethyl-salicylate; sodium ethylmercurithiosalicylate; C12-C15 alkyl salicylates; isopropylbenzyl salicylate; zinc glycinate salicylate; and mixtures thereof.

In an embodiment of the invention, a salicylic acid derivative may be selected from metal salicylates; alkyl salicylates of chain length C10 or greater; betaine salicylates; TEA-salicylate; MEA-salicylate; and mixtures thereof.

In an embodiment, the active substance in the formulation is selected from salicylic acid, metal salicylates and mixtures thereof. In an embodiment, it is salicylic acid.

In an embodiment, the active substance is not a salicylanilide, for example a halogenated salicylanilide of the type referred to in GB-2 456 376. In an embodiment, the active substance is not a C1 to C9 alkyl salicylate, or is not a C1 to C9 alkyl salicylate which is the same as component (ii) of the solvent system.

In an embodiment, a derivative of salicylic acid is acceptable for pharmaceutical (which includes veterinary) and/or cosmetic use.

The salicylic acid (or any derivative thereof) may be either naturally or synthetically derived. It may for example be obtained from a natural source such as willow herb. The concentration of the salicylic acid or derivative in a formulation according to the invention might suitably be 0.05 or 0.1 or 0.2 or 0.5% w/w or greater, for example 0.8% w/w or greater or in cases 0.9 or 1 or 1.5 or even 2% w/w or greater. Its concentration might be up to 10% w/w, or up to 5 or 3 or 2% w/w, or in cases up to 1.5 or 1.2 or 1% w/w. In an embodiment of the invention, the concentration of the salicylic acid or derivative is from 0.5 or 1 to 5% w/w, or from 0.5 or 1 to 3% w/w, or from 0.5 or 1 to 2% w/w, such as about 1 or 2% w/w. In cases, for example in chemical peels for use against acne, its concentration might be up to 10 or 20 or even 25 or 30% w/w.

The concentration of the DMI in the formulation may be 10% w/w or greater, or 15 or 20% w/w or greater, or 22 or 25 or 26 or 27 or 28% w/w or greater, or in cases 30 or 35% w/w or greater. Its concentration may be up to 35% w/w, or up to 32 or 30 or 29% w/w. In cases it may be up to 40 or even 45% w/w. Its concentration may for example be from 25 to 35% w/w, or from 20 to 30% w/w, or from 25 to 30% w/w, or from 27 to 30 or 33% w/w, such as about 30% w/w. In another embodiment it may be present at about 40% w/w.

The component (ii) is suitably a liquid. It may be an ester of salicylic acid with a suitable alcohol. It may be a C3 to C9 alkyl salicylate or a C5 to C9 alkyl salicylate. The alkyl group may be straight chain or branched, and/or may incorporate one or more cyclic groups. The component (ii) may for example be selected from amyl salicylate, octyl (ethylhexyl) salicylate, homosalate and mixtures thereof. It may in particular be homosalate, which is the ester formed from salicylic acid and 3,3,5-trimethyl cyclohexanol. The concentration of the component (ii) in the formulation may be 5% w/w or greater, or 6 or 7 or 8 or 9 or 9.5% w/w or greater. Its concentration may be up to 13% w/w, or up to 12 or 11 or 10% w/w. Its concentration may for example be from 5 to 10% w/w, or from 6 to 13% w/w, or from 9 to 11% w/w, such as about 9.6 or 10% w/w. In an embodiment, its concentration may for example be from 5 to 12% w/w, or from 6 to 11% w/w, or from 7 to 10% w/w, such as about 8.5% w/w.

The component (iii) is suitably a liquid. It may be for example a glyceryl mono-, di- or tri- fatty acid ester. In one embodiment it is a monoester; in another it is a diester. It may be an ester of a C12 to C22 fatty acid, or of a C12 to C20 fatty acid, or of a C12 to C18 fatty acid, such as a glyceryl oleate or stearate. It may be an L-pyrrolidone carboxylic acid (PCA) glyceryl ester, for example of a C12 to C22 or C12 to C20 or C12 to C18 (in particular C18) fatty acid, for example PCA glyceryl oleate. It may be glyceryl diisostearate. In an embodiment it may be a diglyceride ester, in particular a diglyceride mono- or diester. In an embodiment it may be a polyglyceride ester, in particular a polyglyceride mono- or diester. In an embodiment, it is selected from PCA glyceryl esters (in particular PCA glyceryl oleate), glyceryl diisostearate, and mixtures thereof.

The concentration of the component (iii) in the formulation may be 2% w/w or greater, or 3 or 3.5 or 4 or 4.5 or 5% w/w or greater. Its concentration may be up to 12% w/w, or up to 10 or 8% w/w, or up to 7 or 6.5 or 6 or 5.5 or 5% w/w. Its concentration may for example be from 3 to 7% w/w, or from 4 to 6% w/w, such as about 5% w/w. Where the formulation comprises a mixture of two or more components (iii), the above concentration ranges suitably apply to the overall mixture. Thus, for example, the formulation may contain from 3 to 7% w/w of a first glyceryl fatty acid ester, such as PCA glyceryl dioleate, and from 3 to 7% w/w of a second glyceryl fatty acid ester, such as glyceryl diisostearate.

In an embodiment, in particular although not exclusively when the component (iii) is glyceryl diisostearate, the concentration of the component (iii) in the formulation may be up to 45% w/w, or up to 40% w/w, or up to 35 or 30 or 25 or 20% w/w. In such a case its concentration may for example be 15% w/w or greater, or 18 or 20% w/w or greater, such as from 15 to 40% w/w or from 20 to 40% w/w, in cases about 18 to 22% w/w or about 38 to 40% w/w. A lower concentration of component (iii) may be appropriate in formulations which also contain an alcohol, and/or which contain one or more of the optional components (v) to (viii). Thus, in certain embodiments it may be preferred for the concentration of glyceryl diisostearate in the formulation to be 8% w/w or less, or 7 or 6 or 5% w/w or less, or in cases to be 4 or 3 or 2% w/w or less. In some embodiments, it may be preferred for the formulation not to contain any glyceryl diisostearate.

Where the solvent system comprises PCA glyceryl oleate, it may be preferred that its concentration in the overall formulation be 8% w/w or less, or 7 or 6 or 5% w/w or less, or in cases 4 or 3 or 2% w/w or less. In some embodiments, it may be preferred for the formulation not to contain any PCA glyceryl oleate. In some embodiments, it may be preferred for the formulation not to contain both glyceryl diisostearate and PCA glyceryl oleate.

The alcohol component (iv), if present, may for example be selected from C1 to C3 alcohols such as ethanol and isopropyl alcohol; phenoxyethanol; 1-methoxy-2-propanol; benzyl alcohol; THFA; Transcutol™ (2-(2-ethoxyethoxy)ethanol); and mixtures thereof, or from C1 to C3 alcohols such as ethanol; THFA; phenoxyethanol; benzyl alcohol; and mixtures thereof. In an embodiment the alcohol is a C1 to C3 alcohol, in particular ethanol. In an embodiment it is THFA. In an embodiment it is selected from ethanol, THFA and in particular mixtures thereof.

The concentration of the component (iv) in the formulation, if present, may be 5 or 8 or 10% w/w or greater, or 15% w/w or greater, or 17 or 18 or 19 or 20% w/w or greater. Its concentration may be up to 35 or 30% w/w, or up to 25 or 23 or 22 or 21 or 20% w/w. Its concentration may for example be from 10 to 20% w/w, or from 15 to 25% w/w, or from 15 or 18 to 22% w/w, such as about 19 or 20% w/w. In an embodiment, its concentration is from 5 to 15% w/w, such as about 10% w/w.

Where the formulation comprises a mixture of two or more alcohol components (iv), the above concentration ranges suitably apply to the overall mixture. It may for example contain from 5 to 20% w/w of a first alcohol such as THFA, together with from 2 to 15% w/w of a second alcohol such as ethanol. In an embodiment, it contains from 5 to 15% w/w, or from7 to 13% w/w, of THFA, together with from 5 to 15% w/w, or from 7 to 13% w/w, of ethanol. In some embodiments, it may be preferred for the THFA concentration to be less than 15% w/w, for example 14 or 13 or 12% w/w or less.

In an embodiment, where the solvent system comprises ethanol, the concentration of the ethanol in the overall formulation may be greater than 5% w/w, or greater than 6 or 7 or 8% w/w. However, it may also be preferred to maintain the ethanol concentration at 15% w/w or lower, in particular at 10% w/w or lower, for example at 9 or 8 or 7% w/w or lower.

In an embodiment, where the solvent system comprises THFA, the concentration of the THFA in the overall formulation may be 20% w/w or less, or 17.5 or 15% w/w or less, such as 12.5 or ideally 10% w/w or less.

Where the formulation contains one or more of the optional components (vi) to (viii), in particular two or more of those components, and more particularly all three, it may be preferred for the formulation not to contain the alcohol (iv).

The component (v), if present, is suitably a liquid. In an embodiment, it is a polyoxyalkylene (polyalkoxylated) glyceryl ester, in particular a polyoxyethylene (polyethylene glycol, PEG) glyceryl ester. In an embodiment, it is a polyalkylene glycol, in particular a PEG. It suitably has a molecular weight of 350 or greater, or of 380 or 400 or 450 or greater, for example of 500 or 600 or 700 or 800 or greater.

A polyalkoxylated glyceryl ester (also known as a polyalkoxylated glyceride) is a glyceride ester of a polyalkylene glycol (typically PEG). It may contain mono-, di- or tri-glycerides, partial glycerides or mixtures thereof. It may for example be formed by esterification of a polyalkylene glycol with a glyceride oil of an appropriate chain length. The glyceride components may for example contain from 6 to 20 or from 8 to 18 carbon atoms; they may be selected from caprylic and capric glycerides and mixtures thereof. Suitable such solvents are commercially available as Glycerox™ 767 and Labrasol™, both of which are mixtures of caprylic and capric glycerides of PEGs.

A polyethoxylated glyceryl ester usable as a component (v) in the present invention may be a mixture of PEG-8 caprylic and capric glycerides, such as is commercially available as Labrasol™.

In an embodiment, the component (v) may be or comprise a polyethylene glycol (PEG), suitably a PEG having a molecular weight of 350 or greater, or of 380 or 400 or 450 or greater, for example of from 350 to 450. A suitable example might be a PEG having a molecular weight of about 400.

In an embodiment, the solvent system contains a mixture of two or more components (v). In an embodiment, the component (v) is selected from polyalkoxylated glyceryl esters, polyalkylene glycols (in particular PEGs), and mixtures thereof.

The concentration of the component (v) in the formulation, if present, may be 6% w/w or greater, or 7 or 8 or 9 or 9.5% w/w or greater. Its concentration may be up to 15 or 13% w/w, or up to 12 or 11 or 10% w/w. Its concentration may for example be from 6 to 13% w/w, or from 8 or 9 to 11% w/w, such as about 9 or 10% w/w.

In an embodiment, the component (v) is present in the formulation at up to 40% w/w, or more particularly at up to 35% w/w, or up to 30% w/w, for example from 20 to 30% w/w or from 22 to 32% w/w or from 25 to 30% w/w, such as about 27% w/w. In an embodiment, it is present in the formulation at up to 25% w/w, or up to 20% w/w, such as about 14 or 18% w/w. A lower concentration of component (v) may be appropriate in formulations which also contain one or more of the optional components (vi) to (viii).

Where the formulation comprises a mixture of two or more components (v), the above concentration ranges suitably apply to the overall mixture. It may for example contain from 10 to 30% w/w or from 15 to 30% w/w of a first component (v) such as a polyethoxylated glyceryl ester (eg Labrasol™), together with from 1 to 10% w/w of a second component (v) such as a PEG.

The component (vi), if present, is suitably a liquid. It may for example be selected from C1 to C12 alkyl pyrrolidones such as ethyl pyrrolidone; caprylyl pyrrolidone; lauryl pyrrolidone; and mixtures thereof. In an embodiment, it is a C1 to C4 alkyl pyrrolidone, or a C1 to C3 or C1 to C2 alkyl pyrrolidone, in particular ethyl pyrrolidone. In an embodiment, it is 2-pyrrolidone. The concentration of the component (vi) in the formulation, if present, may be 13% w/w or greater, or 15 or 16 or 17 or 18 or 18.5 or 19% w/w or greater. Its concentration may be up to 25% w/w, or up to 24 or 23 or 22 or 21 or 20 or 19.5% w/w. Its concentration may for example be from 15 to 25% w/w, or from 18 to 21% w/w or from 18 to 20% w/w, such as about 20% w/w. In an embodiment, it may be preferred for the formulation not to contain the component (vi).

The component (vii), if present, is suitably a liquid. It may be a C1 to C4 alkyl glucose mono-, di- or triester of a C12 to C18 (in particular C18) fatty acid. It may be a C1 to C4 alkyl glucose diester of a C12 to C18 (in particular C18) fatty acid, for example a C1 to C4 alkyl glucose dioleate such as methyl glucose dioleate. It may be a C1 to C2 alkyl glucose ester, for example a methyl glucose ester (which includes a methyl glucose diester). The concentration of the component (vii) in the formulation, if present, may be 2% w/w or greater, or 3 or 3.5 or 4 or 4.5 or 5% w/w or greater. Its concentration may be up to 8% w/w, or up to 7 or 6.5 or 6 or 5.5 or 5% w/w. Its concentration may for example be from 3 to 7% w/w, or from 4 to 5.5% w/w, such as about 4.8 or 5% w/w.

The component (viii), if present, is suitably a liquid. It may be for example methyl pyruvate or ethyl pyruvate, in particular ethyl pyruvate. Its concentration in the formulation may be 5% w/w or greater, or 7 or 10 or 13 or 14% w/w or greater, or in cases 15 or 17 or 18 or 18.5% w/w or greater. Its concentration may be up to 25% w/w, or up to 22 or 20 or 19.5% w/w, or in cases up to 18 or 16 or 15% w/w. Its concentration may for example be from 15 to 25% w/w, or from 18 to 20% w/w, such as about 19 or 19.1% w/w. In an embodiment, its concentration may be from 10 to 20% w/w, or from 12 to 17% w/w, or from 12 or 13 to 16 or 14 to 15% w/w, such as about 13.5 or 14.5% w/w.

In an embodiment, a component (viii), in particular ethyl pyruvate, may be combined with an additional solvent such as THFA, for instance in a 50:50 v/v mixture, the concentration of such a mixture being typically as described above for the component (viii) alone. In an embodiment, it may be preferred for the formulation not to contain the component (viii).

The formulation of the invention should be suitable for topical application to the skin, in particular human skin. It may be adapted and/or intended for topical application to the skin. The formulation may be suitable and/or adapted and/or intended for use as a pharmaceutical formulation, or as a cosmetic. In an embodiment it is suitable and/or adapted and/or intended for use as an anti-acne preparation, and/or as a keratolytic preparation. It may be suitable and/or adapted and/or intended for use in the treatment of one or more of the conditions identified below in connection with the fourth aspect of the invention.

Any component (i) to (viii) included in the formulation should therefore be suitable for topical application to the skin. It should not induce unacceptable levels of irritation on application to the skin, ideally even when the formulation is applied as a "leave-on" treatment. Ideally, such components are acceptable for cosmetic use. Ideally they are acceptable for pharmaceutical (which includes veterinary) use, in particular for pharmaceutical administration to humans.

In an embodiment, the formulation does not contain water, or contains less than 5 or 2 or 1 or 0.5 or 0.1% w/w water. It may for example take the form of a non-aqueous gel. In an embodiment, it does not contain propylene glycol, or contains less than 5 or 2 or 1 or 0.5 or 0.1% w/w propylene glycol. In an embodiment, it does not contain ethanol, or contains less than 10 or 8 or 5 or 2 or 1 or 0.5 or 0.1% w/w ethanol.

The salicylic acid or derivative thereof should be soluble in the solvent system, by which is meant that the solvent system is able to dissolve the salicylic acid or derivative to at least 0.01% w/w, or at least 0.05 or 0.1% w/w, or at least 0.25 or 0.5% w/w, for example to 0.8% w/w or greater or in cases 1 or 1.5 or 2 or 3 or 4% w/w or greater. In an embodiment, the solvent system is able to dissolve the salicylic acid or derivative thereof to at least 1% w/w.

In an embodiment, in particular where it is for use in the treatment of acne, the formulation of the invention may contain usnic acid or an usnate. Usnic acid and usnate salts are known for use as topical antimicrobial agents. Some, for example copper usnate, have been found to be active against *P. acnes,* the bacteria implicated in inflammatory acne, and have thus been suggested for use as topical anti-acne agents.

The usnic acid or usnate salt may be present in the formulation as an active substance. It is typically present as an antimicrobial agent, in particular as an antibacterial agent, more particularly as an agent against propionibacteria such as *P. acnes,* staphylococci such as *S. aureus,* and/or coryneform bacteria. It may be present as an anti-acne agent.

An usnate salt may be a metal salt. It may be an alkali metal salt such as sodium usnate or an alkaline earth metal salt such as calcium di-usnate, in particular the former. It may be a salt of a multivalent, in particular divalent, metal such as copper (II): it may therefore be a di-usnate, for example copper (II) di-usnate.

In a specific embodiment of the invention, the usnate salt is selected from copper (II) di-usnate, sodium usnate and mixtures thereof. In another specific embodiment, the usnate salt is copper (II) di-usnate (referred to hereafter simply as "copper usnate").

The concentration of the usnic acid or usnate salt, if present in the formulation, may be 0.01% w/w or greater, or 0.05 or 0.1% w/w or greater, or 0.25 or 0.5% w/w or greater, for example 0.8% w/w or greater or in cases 0.9 or 1% w/w or greater. Its concentration may be up to 5% w/w, or up to 3 or 2% w/w, or up to 1.5 or 1.2 or 1% w/w. In an embodiment, its concentration is from 0.1 to 2% w/w, or from 0.5 to 2% w/w, or from 0.5 to 1.5% w/w, such as about 1% w/w.

In an embodiment, the formulation of the invention contains a C12 to C20 fatty acid or mixture thereof. It may contain a C12 to C18 fatty acid or mixture thereof, or a C14 to C18 fatty acid or mixture thereof, for example selected from caprylic/capric, lauric, palmitic, stearic, sapienic, arachidic, oleic and linoleic acids and mixtures thereof. One or more of the fatty acids may be a constituent of sebum, in particular human sebum: such acids include palmitic acid, sapienic acid and oleic acid, and others described by Nicolaides N in "Skin Lipids - Their Biochemical Uniqueness", Science, 1974, 186: 19-26. In an embodiment, the formulation contains oleic acid. The concentration of such a component in the formulation may be 0.05% w/w or greater, for example 0.1 or 0.2 or 0.3 or 0.4 or 0.5% w/w or greater. Its concentration may be up to 5% w/w, or up to 2 or 1% w/w, for example up to 0.9 or 0.8 or 0.7 or 0.6% w/w. Its concentration may be for example from 0.3 to 0.7% w/w, such as about 0.5 or 0.57% w/w. In certain embodiments, however, it may be preferred for the formulation not to contain a C12 to C20 fatty acid.

In an embodiment, the formulation contains L-pyrrolidone carboxylic acid (PCA). The concentration of the PCA in the formulation may be 0.05% w/w or greater, for example 0.1 or 0.2 or 0.3 or 0.4 or 0.5% w/w or greater. Its concentration may be up to 2 or 1.5 or 1% w/w, for example up to 0.9 or 0.8 or 0.7 or 0.6% w/w. Its concentration may be for example from 0.2 to 1.3% w/w, or from 0.3 to 0.7% w/w, or from 0.7 to 1.3% w/w, such as about 0.5% w/w or about 1% w/w. In certain embodiments, however, it may be preferred for the formulation not to contain PCA.

A formulation according to the invention may also contain an antioxidant. This can help to stabilise the active substance(s) present, which may be unstable in light and/or susceptible to oxidation in some solvent environments. Oxidative stability can be particularly important for formulations which are intended for use as "leave-on" products, since they can remain on the skin, exposed to both oxygen and sunlight, for extended periods. Antioxidants suitable for use in topical skin treatment formulations are well known to those skilled in the art.

Where the formulation contains an antioxidant, its concentration may be 0.1% w/w or greater, or 0.2 or 0.3 or 0.4 or 0.5% w/w or greater, or in cases 0.6 or 0.7 or 0.8% w/w or greater. Its concentration may be up to 2 or 1.5% w/w, or up to 1.4 or 1.3 or 1.2% w/w, such as about 1% w/w.

The formulation may contain one or more additional ingredients or excipients, as are known for use in topical skin treatment formulations. Those included will depend on the intended mode of application for the formulation. Examples may for instance be found in Williams' Transdermal and Topical Drug Delivery (Pharmaceutical Press, 2003) and other similar reference books. See also the Database of Cosmetic and Toiletry Formulations (CD ROM, 2005) by Ernest W Flick, published by William Andrew; the Personal Care Product Council's online database (www.ctfa-online.org); and approved ingredients lists published by regulatory authorities, for example the EU Cosmetic Ingredients list which is available online through the European Commission (see http://ec.europa.eu/enterprise/cosmetics/cosing).

Suitable additives may for instance include emollients, moisturisers, preservatives, stabilisers, gelling agents and other thickeners, sunscreens, colouring agents and fragrances. For use in the treatment of acne, however, it may be preferred for the formulation not to contain an emollient. In a specific embodiment, the formulation contains a fragrance, for example an essential oil or component thereof such as vanillin. In general terms, it may include a component capable of masking, at least partially, the smell of another component present in the formulation.

In an embodiment, the formulation contains one or more thickening agents, which may be gelling agents. Suitable such agents include cellulose-based thickening agents such as methyl cellulose, ethyl cellulose, hydroxyethyl cellulose, hydroxypropyl cellulose, hydroxypropyl methyl cellulose and carboxymethyl celluloses. Such agents may be used in the form of a (preferably cosmetically and/or pharmaceutically acceptable) salt such as for instance the sodium salt. A thickening agent may be a polymeric thickening agent such as a carbomer, which will typically be a cross-linked acrylic acid-based polymer, for example a homopolymer of acrylic acid cross-linked with an allyl ether: such thickeners are available for instance in the Carbopol™ range, ex-Lubrizol.

In an embodiment, the formulation contains a hydroxypropyl cellulose thickening agent, for example of the type commercially available as Klucel™ from Hercules, USA. Such a thickening agent may for example have a molecular weight of 200,000 or greater, or of 300,000 or 350,000 or greater, for example from 300,000 to 500,000 or from 300,000 to 400,000.

Where the formulation contains a thickening agent, its concentration may be 0.2% w/w or greater, or 0.5% w/w or greater, or 1 or 1.5% w/w or greater, or in cases 5%w/w or greater. Its concentration may be up to 10% w/w, or more usually up to 5% w/w, or up to 4 or 3 or 2.5 or 2% w/w, for example from 1 to 2.5% w/w or from 1.5 to 2.5% w/w or from 1.5 to 3% w/w. In an embodiment, the concentration of the thickening agent in the formulation is about 1.9 or 2% w/w, or about 2.5% w/w. In cases it may be used at 1% w/w or less. However, a suitable concentration for a thickening agent (or mixture thereof) will depend on the desired viscosity of the ultimate formulation, and on the properties of the thickening agent as well as any restrictions on its permitted levels in for example cosmetic or pharmaceutical preparations.

The formulation may contain one or more additional active agents such as antimicrobial (in particular antibacterial) agents. For example, it may contain one or more agents selected from anti-acne agents, keratolytics, comedolytics, agents capable of normalising keratinocyte and/or sebocyte function, anti-inflammatories, anti-proliferatives, antibiotics, anti-androgens, sebostatic/sebosuppressive agents, anti-pruritics, immunomodulators, agents which promote wound healing, additional antimicrobial agents, anti-perspirant agents, deodorants, and mixtures thereof.

An additional antimicrobial agent may be selected from biocides, disinfectants, antiseptics, antibiotics, bacteriophages, enzymes, anti-adhesins, immunoglobulins, antimicrobially active antioxidants, and mixtures thereof; it may be active as a bactericide, in particular against propionibacteria. It may however be preferred for the salicylic acid or derivative (and where appropriate the usnic acid or usnate) to be the only active agent(s) in the formulation, or at least to be the only antimicrobially or antibacterially active agent(s) and/or the only anti-acne active agent(s) and/or the only keratolytically active agent(s).

In a specific embodiment, a formulation according to the invention contains salicylic acid or a derivative thereof dissolved in a solvent system, wherein the solvent system comprises:
(1) DMI, suitably at a concentration from 25 to 35% w/w or from 25 to 30% w/w;
(2) a C5 to C9 alkyl salicylate, in particular homosalate, suitably at a concentration from 5 to 12% w/w;
(3) a glyceryl fatty acid ester selected from PCA glyceryl oleate, glyceryl diisostearate and mixtures thereof, suitably at a concentration from 2 to 8% w/w;
(4) an alcohol selected from ethanol, THFA and mixtures thereof, suitably at a concentration from 15 to 25% w/w;
(5) a component (v) selected from polyoxyethylene glyceryl esters (in particular Labrasol™), PEGs, and mixtures thereof, suitably at a concentration from 22 to 32% w/w; and
(6) a methyl glucose ester, in particular methyl glucose dioleate, suitably at a concentration from 2 to 8% w/w.

Such a formulation suitably also comprises a cellulosic thickening agent, in particular hydroxypropyl cellulose, suitably at a concentration from 1 to 4% w/w. The concentration of the salicylic acid or derivative in such a formulation is suitably from 0.5 to 1.5% w/w.

Such a formulation suitably comprises usnic acid or an usnate salt (in particular an usnate such as copper usnate), suitably at a concentration from 0.5 to 1.5% w/w. It suitably has the form of a gel.

The formulation of the invention may be in the form of a fluid, for example a lotion, cream, ointment, varnish, paste, gel or other viscous or semi-viscous fluid, or a less viscous fluid such as might be used in sprays, foams, drops and dropping fluids, or aerosols. A liquid formulation may itself be formulated as suspended (for example aerosolised) liquid droplets within another fluid carrier.

The formulation may in particular be in the form of a solution, lotion, cream or gel. In an embodiment, it is a cream or gel, in particular a gel. It may comprise a formulation which is, or may be, applied to a carrier such as a sponge, swab, brush, pad, tissue, cloth, wipe, skin patch, dressing (or other material designed for application to the skin), to facilitate its topical administration.

A formulation according to the invention may be incorporated into, and hence applied in the form of, another product such as a hair care or in particular a skin care preparation (for example a skin cleanser, toner or moisturiser, or a shampoo); a deodorant or anti-perspirant; a cosmetic (eg a makeup product); a cleansing preparation (for example a hand, body or facial wash); a pharmaceutical (which includes veterinary) preparation; a cosmeceutical preparation; or a toiletry product (for instance a bath or shower additive, a soap or a skin scrub). The formulation may for example be, or be incorporated into, a wash-off skin treatment product such as a skin cleanser, or in particular a leave-on skin treatment product.

The invention thus provides, according to a second aspect, a product which incorporates a topical skin treatment formulation according to the first aspect.

A third aspect of the invention provides a method for preparing a topical skin treatment formulation according to the first aspect, the method involving dissolving salicylic acid or a derivative thereof in a solvent system of the type defined above with reference to the first aspect of the invention. The components of the formulation may be mixed together in conventional manner. For example, the salicylic acid or derivative may firstly be dissolved in one or more of the components in which it is relatively freely soluble, prior to mixing with any components in which it is less freely soluble and any other remaining ingredients of the formulation (for example thickeners, fragrances and/or antioxidants). Stirring and/or heating may be used to aid efficient mixing of ingredients, and/or dissolution of the salicylic acid or derivative, at appropriate stages during such a process.

According to a fourth aspect of the invention, there is provided a formulation according to the first aspect, for use in a method of therapy carried out on a living human or animal, in particular human, body. In this context the solvent system should be pharmaceutically acceptable (which includes acceptable for veterinary use).

In an embodiment, the formulation is for use in the treatment of a condition (in particular a skin or skin structure condition) which is amenable to treatment by a keratolytic agent. Such conditions include for example acne, dandruff, psoriasis, calluses, corns, keratosis pilaris and warts. In a specific embodiment, the formulation is for use in the treatment of acne.

In some embodiments, in particular when the formulation contains usnic acid or an usnate, it may be for use in the treatment of a condition which is caused by, transmitted by and/or exacerbated by (in particular either caused or transmitted by, more particularly caused by) microbial activity. The microbial activity may be bacterial or fungal activity, in particular bacterial activity, more particularly propionibacterial activity.

The formulation may be for use in the treatment of a condition which affects, or is associated with, follicles such as pilosebaceous follicles. For example, the formulation may be for use against a micro-organism which is present in the follicles, such as a staphylococcus or in particular a propionibacterium such as *P. acnes, P. granulosum* or *P. avidum.* It may be for use in the treatment of a condition which affects, or is associated with, the stratum corneum: the formulation may therefore be for use against micro-organisms which are present in the stratum corneum, for example staphylococci or coryneform bacteria, and/or it may be for use against a condition which is amenable to keratolytic treatment of the stratum corneum.

In an embodiment of the fourth aspect of the invention, the formulation is for use in the treatment of a skin or skin structure condition. Such a condition may be a primary or secondary infection. It may for example be a superficial or uncomplicated skin infection amenable to local therapy. It may be caused, transmitted and/or exacerbated by a Gram-positive bacterium such as a staphylococcus or propionibacterium. It may be acne or an infection associated with acne. It may be a primary or secondary infection due to *S. aureus* (including MRSA) or a group A beta haemolytic streptococcus. Other skin and skin structure conditions which might be treated according to the invention include infected atopic eczema, superficial infected traumatic lesions, wounds, burns, ulcers, impetigo and folliculitis.

In an embodiment, the formulation is for use in the treatment of acne. Acne is a multifactorial disease of the pilosebaceous follicles of the face and upper trunk, characterised by a variety of inflamed and non-inflamed lesions such as papules, pustules, nodules and open and closed comedones. Its treatment can therefore encompass the treatment (which embraces prevention or reduction) of any of these symptoms, and references to use as an anti-acne agent may be construed accordingly. In particular, the treatment of acne encompasses the treatment (including prevention) of lesions and/or scarring associated with acne. It also encompasses the inhibition of propionibacterial activity which could cause or be otherwise associated with acne or its symptoms. In the context of the present invention, it may be or involve the treatment of non-inflamed acne lesions. Instead or in addition, it may be or involve the treatment of inflamed acne lesions.

In the context of the present invention, treatment of a condition encompasses both therapeutic and prophylactic treatment, of either an infectious or a non-infectious condition. It may involve complete or partial eradication of the condition, removal or amelioration of associated symptoms, arresting subsequent development of the condition, and/or prevention of, or reduction of risk of, subsequent occurrence of the condition. It will typically involve use of the formulation as a keratolytic and/or anti-acne agent. It may involve use of the formulation as an antimicrobial (in particular antibacterial) agent.

Treatment may involve use of the formulation to treat a condition which is caused, transmitted and/or exacerbated by (in particular caused or transmitted by, more particularly caused by), or which is associated with, microbial (in particular bacterial) biofilm formation.

The treatment will be administered topically. It may for example involve applying the formulation to the skin daily, or in particular twice daily, as a leave-on formulation. For the treatment of acne, the formulation may be applied to the entire face, or to one or more regions thereof.

A fifth aspect of the invention provides the formulation according to the first aspect, for use in the treatment of a condition (in particular a skin or skin structure condition) which is amenable to treatment by a keratolytic agent, or of a condition which is caused by, transmitted by and/or exacerbated by (in particular either caused or transmitted by, more particularly caused by) microbial activity. The condition may be selected from those listed above in connection with the first to the fourth aspects of the invention. It may in particular be acne.

A sixth aspect provides a product according to the claims for use in the treatment of a human or animal patient suffering from or at risk of suffering from a condition (in particular a skin or skin structure condition) which is amenable to treatment by a keratolytic agent, or from a condition which is caused by, transmitted by and/or exacerbated by (in particular either caused or transmitted by, more particularly caused by) microbial activity, the method involving administering to the patient a therapeutically (which term includes prophylactically) effective amount of a formulation according to the first aspect. The formulation may be administered in a keratolytically and/or antimicrobially effective amount. It should be administered topically.

In an embodiment of the sixth aspect of the invention, the patient is suffering from the relevant condition. In an embodiment, the patient is a human patient. Again, the condition may be selected from those referred to above in connection with the first to the fourth aspects of the invention.

A seventh aspect provides the use of a formulation according to the first aspect of the invention, for non-therapeutic purposes. In an embodiment of this seventh aspect, the formulation is used as a skin care agent for non-therapeutic purposes, for example for cosmetic purposes such as to improve the appearance, feel or smell of the skin. It may be used as a toiletry or makeup product.

According to an eighth aspect of the invention, there is provided a formulation according to the first aspect, and/or of a solvent system as defined in connection with the first aspect, for the purpose of improving the delivery of salicylic acid or a derivative thereof (in particular salicylic acid itself) to a target site in or on the skin. In this context, improving the delivery of the salicylic acid or derivative may involve increasing the rate of its delivery, or the rate of onset of - or persistence of - its activity at the target site; increasing the amount or proportion of it which reaches the target site, or the amount or proportion which reaches the target site within a specified time period, or which remains at the target site after a specified time period; increasing control over the rate or time or quantity of delivery or the rate of onset or persistence of activity; and/or targeting the delivery more accurately to the target site or to a desired delivery time. Improving the delivery of the salicylic acid or derivative may involve enhancing the efficacy or the perceived efficacy of the salicylic acid or derivative at the target site, which may involve increasing the speed and/or magnitude and/or duration and/or locus of the effect (typically a therapeutic effect, for example an antimicrobial and/or keratolytic and/or anti-acne effect) that the salicylic acid or derivative has at the target site, or increasing control over the speed, magnitude, timing, duration or locus of the effect. The invention may be used to achieve any degree of improvement in the delivery of the salicylic acid or derivative. The target site may in particular be the stratum corneum and/or the pilosebaceous follicles.

A formulation according to the invention may be marketed with an indication that it provides improved delivery of the salicylic acid or derivative contained within it. Such marketing may include an activity selected from (a) enclosing the formulation in a container or package that comprises the relevant indication; (b) packaging the formulation with a package insert that comprises the indication; (c) providing the indication in a publication or sign that describes the formulation; and (d) providing the indication in a commercial which is aired for instance on the radio, television or internet. The improved delivery may be attributed, in such an indication, at least partly to the nature of the solvent system in which the salicylic acid or derivative is dissolved. The invention may involve assessing the delivery of the salicylic acid or derivative from the formulation during or after its preparation. It may involve assessing the delivery of the salicylic acid or derivative both in the presence and the absence of the solvent system required by the present invention, for example so as to confirm that the solvent system contributes to improved delivery.

Throughout the description and claims of this specification, the words "comprise" and "contain" and variations of the words, for example "comprising" and "comprises", mean "including but not limited to", and do not exclude other moieties, additives, components, integers or steps. Moreover the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects. Other features of the invention will become apparent from the following examples. Generally speaking the invention extends to any novel one, or any novel combination, of the features disclosed in this specification (including any accompanying claims and drawings). Thus features, integers, characteristics, compounds, chemical moieties or groups described in conjunction with a particular aspect, embodiment or example of the invention are to be understood to be applicable to any other aspect, embodiment or example described herein unless incompatible therewith. Moreover unless stated otherwise, any feature disclosed herein may be replaced by an alternative feature serving the same or a similar purpose.

Where upper and lower limits are quoted for a property, for example for the concentration of a component in a formulation, then a range of values defined by a combination of any of the upper limits with any of the lower limits may also be implied.

In this specification, references to properties such as solubilities, liquid phases and the like are - unless stated otherwise - to properties measured under ambient conditions, ie at atmospheric pressure and at a temperature of from 18 to 25°C, for example about 20°C.

The present invention will now be further described with reference to the following non-limiting examples and the accompanying illustrative figures, of which:
Figures 1 to 6 are graphs showing weights of stratum corneum removed at various time points, during the skin sampling experiments described in Example 2 below; and
Figures 7 to 12 are plots of mean cumulative SA quantities against mean cumulative SC quantities, during the skin sampling experiments of Example 2.

### Detailed description

These experiments involved preparing topical skin treatment formulations according to the invention, and testing their effects in vivo.

### Example 1 - formulation A

A gel formulation A according to the invention was prepared using the components and concentrations listed in Table 1. Copper usnate was present as an antibacterial agent, known to be active against the propionibacteria which are implicated in inflammatory acne. The first seven components together represented the solvent system in which the salicylic acid and copper usnate actives were dissolved.

**Table 1 - Formulation A**

| ***Component*** | ***Concentration (% w*/*w)*** | ***Source*** |
|---|---|---|
| Dimethyl isosorbide (DMI) | 30 | Sigma-Aldrich, UK |
| Ethyl pyrrolidone | 20 | Sigma-Aldrich, UK |
| Ethyl pyruvate | 13.5 | Sigma-Aldrich, UK |
| Homosalate | 10 | TCI Europe |
| PEG-8 caprylic/capric glycerides | 10 | Gatefosse |
| Methyl glucose dioleate | 5 | Surfachem Group Ltd |
| PCA glyceryl oleate | 5 | Dr Straetmans Chemische Produkte GmbH |
| Salicylic acid | 2 | Sigma-Aldrich, UK |
| Copper usnate | 1 | Variati |
| Antioxidant (ascorbyl | 1 | Sigma-Aldrich, UK |
| palmitate) | | |
| Thickener (hydroxypropyl cellulose) | 2 | Honeywill & Stein Ltd |
| Oleic acid | 0.5 | Sigma-Aldrich, UK |
| TOTAL | 100.0 | |

| | | |
|---|---|---|
| PCA = L-pyrrolidone carboxylic acid | | |

The formulation was prepared as follows. The copper usnate was premixed with the DMI, the ethyl pyrrolidone, the ethyl pyruvate and the homosalate. The mixture was heated and stirred to optimise usnate dissolution, then filtered to remove any undissolved residue. The glyceryl fatty acid ester was then added, along with the remaining components of the solvent system and the oleic acid, and mixed vigorously. To this mixture was added the salicylic acid and antioxidant, followed by the thickener. The resultant mixture was stirred and heated, shaken vigorously and then left to gel for 2 hours at room temperature.

### Example 2 - formulation delivery in vivo (formulation A)

This experiment used formulation A as described in Example 1, containing salicylic acid as a keratolytic and anti-acne active substance. It investigated the rate and depth of penetration of the salicylic acid into the stratum corneum; the speed of onset of its keratolytic action; and its persistence in the skin following application. The method used was the so-called "tape stripping" method.

### Tape stripping - background

As discussed above, salicylic acid (SA) is a well established topical treatment for mild to moderate acne, and there are several clinical study reports to support its efficacy (see Roth HL, "Acne vulgaris: evaluation of a medicated cleansing pad", Calif Med 1964, 100(165): 167; Shalita AR, "Treatment of mild and moderate acne vulgaris with salicylic acid in an alcohol-detergent vehicle", Cutis 1981, 28(5): 556-558; and Eady EA et al, "The benefit of 2% salicylic acid lotion in acne - A placebo-controlled study", J Dermatol Treat 1996, 7: 93-96).

The formation of microcomedones, which block the pilosebaceous follicles, is regarded as a key early event in the pathogenesis of acne and there is significant correlation between the severity of acne and the number and size of microcomedones (Cunliffe WJ et al, "Comedone formation: etiology, clinical presentation, and treatment", Clin Dermatol 2004, 22(5): 367-74). It has been shown using the follicular biopsy technique that SA mediates its therapeutic effect by unblocking the pilosebaceous follicles, and reducing the number of microcomedones (Mills OH Jr & Kligman AM, "Assay of comedo lytic activity in acne patients", Acta Derm Venereol, 1983, 63(1): 68-71. It is therefore reasonable to suppose that improved bioavailability of SA will result in improved clinical efficacy.

It is accepted that the keratolytic effect of SA is mediated by loosening the cohesion of layers within the stratum corneum (SC) (Roberts DL et al, "Detection of the action of salicylic acid on the normal stratum corneum", Br J Dermatol 1980, 103(2): 191-6; Waller JM et al, "'Keratolytic' properties of benzoyl peroxide and retinoic acid resemble salicylic acid in man", Skin Pharmacol Physiol 2006, 19(5): 283-9) and that its effect is mediated within the SC (Davies M & Marks R, "Studies on the effect of salicylic acid on normal skin", Br J Dermatol 1976, 95(2): 187-92; Lodén M et al, "Distribution and keratolytic effect of salicylic acid and urea in human skin", Skin Pharmacol 1995, 8(4): 173-8). The mechanism is thought not to involve keratolysis per se: Waller JM et al (see above) have suggested that SA mediates its action via proteolysis of the desmosomes which form tight junctions between keratinocytes (Elias PM et al, "Influence of topical and systemic retinoids on basal cell carcinoma cell membranes", Cancer 1981, 48(4): 932-8; and Hatakeyama S et al, "Retinoic acid disintegrated desmosomes and hemidesmosomes in stratified oral keratinocytes", J Oral Pathol Med 2004, 33(10): 622-8), although this proteolytic activity has yet to be demonstrated experimentally.

However, loosening of SC structure is consistent with earlier histological studies in which other keratolytic or comedolytic agents were applied to experimentally induced comedones in a rabbit ear model (Oh CW & Myung KB, "An ultrastructural study of the retention hyperkeratosis of experimentally induced comedones in rabbits: the effects of three comedolytics", J Dermatol 1996, 23(3): 169-80). Similarly this mechanism is consistent with experiments carried out on normal human skin examining the effects of SA on desquamation and viewed by SEM, in which there was no effect of SA on the mitotic activity of epidermal cells (Davies M & Marks R, see above). Also, pharmacokinetic studies of SA in the SC show that it mediates its effect in six hours, far more rapidly than could be achieved by cellular differentiation and turnover within the epidermis (Lodén M et al, above).

Since SA loosens the binding forces within the stratum corneum, its keratolytic activity can be determined using adhesive tape stripping tests, in which the amount of SC removed at each strip can be measured either by weight or by protein content. The SA removed by each strip can also be determined by extraction and HPLC analysis. There are several reports that have exploited tape stripping to differentiate between SA formulations and/or different keratolytic agents (see Waller JM et al (above); Lodén M et al (above); Tsai J et al, "Distribution of salicylic acid in human stratum corneum following topical application in vivo: a comparison of six different formulations", Int J Pharm 1999 25, 188(2): 145-53; and Bashir SJ et al, "Cutaneous bioassay of salicylic acid as a keratolytic", Int J Pharm 2005, 292(1-2): 187-94). For example, Tsai et al demonstrated statistically significant differences between the total weight of stratum corneum removed (20 strips) among six test formulations, and showed that the most SC was removed with the formulation that delivered the highest concentration of SA to the SC. In a better controlled study Bashir et al (see above) showed unequivocally that formulations containing 2% SA resulted in greater amounts of SC being removed compared to sites that received no SA. In a similarly controlled study Waller JM et al (above) showed that after treatment with benzoyl peroxide greater amounts of SC were removed compared to other treatment and control sites. The results of these reports, together with knowledge of the mode of action of SA, indicate that adhesive tape stripping of normal skin, with analysis of the SC removed, can provide an effective model for comparing the bioavailability of SA delivered in different formulations, and hence the likely therapeutic efficacy of those formulations.

### Tape stripping methodology

The aim in these experiments was to compare the efficacy of two salicylic acid-containing formulations, one a well known market brand and the other a formulation according to the invention. The market brand described itself as a "rapid action treatment cream" and was known to contain 2% w/w salicylic acid. A control (no treatment) was also included in the study, which was laboratory-blinded.

The formulations were applied to opposite forearms of three healthy male volunteers. Skin samples were taken from the subjects using D-squame™ adhesive discs, both before treatment and at a range of subsequent time points. D-Squame™ discs are used in the cosmetics industry for assessing exfoliants: when applied to the skin, they remove with them a layer of stratum corneum cells, which can then be analysed for the amount of stratum corneum removed and also for any active substance (in this case salicylic acid) that has been applied to the skin. Successive samples can be taken from the same skin site at any given time point and the samples analysed to give cumulative figures. This "tape stripping" procedure has been used extensively in dermatological research to evaluate drug delivery from topical formulations.

In this study the D-Squame™ discs were weighed in order to determine the amount of stratum corneum removed. This was used as a measure of the action of salicylic acid, as discussed above. It is also believed to be the case that the more stratum corneum removed, the deeper the stripping process has sampled, and this can be linked to salicylic acid content to give a measure of active penetration. Accordingly, the discs were also solvent extracted and analysed for salicylic acid content. Taken together, these data indicate the bioactivity of the salicylic acid in the test formulations, its speed and depth of penetration from the formulations and its persistence following treatment.

The test formulation was applied evenly, in doses of approximately 0.7g, to three locations on the skin on the forearm of a healthy human volunteer. Skin samples were taken using 22mm CuDerm D-Squame™ skin surface sampling discs (D100, CuDerm), both immediately before the start of the test (T=0), and at 10 minutes, 30 minutes, 1 hour, 2 hours, 4 hours and 24 hours following application of the test formulation. The discs were applied to the test area and pressed down firmly for 2 seconds by depressing the plunger of an inverted 5 ml pipette down onto the disc (equivalent to a pressure of ∼40 kPa). Twenty discs were applied successively to the same skin site at each sampling point, including at T=0.

Each disc was weighed both prior to and immediately after skin sampling, to assess the quantity of stratum corneum removed with it. Three discs which had not been in contact with skin were used as negative controls, and were weighed using the same procedure as for the skin sampling discs.

The weighed discs were stored at 4°C. Their salicylic acid (SA) content was then assessed by HPLC analysis following extraction into methanol. The HPLC sampling conditions were:
- C18 column 150 x 4.6 mm, 5 µm.
- 60:40 isocratic - 0.1% trifluoroacetic acid in water:acetonitrile.
- 1.0 ml/min flow rate.
- Column temperature 25°C.
- 230 nm detection wavelength.
- 20 µl injection volume (50 µg/ml salicylic acid standard in methanol).
- 10 minute run time.

Reference standards of 20.55 and 2.06 µg/ml salicylic acid in methanol were run with each batch of samples, to ensure reproducibility of results.

### Tape stripping results

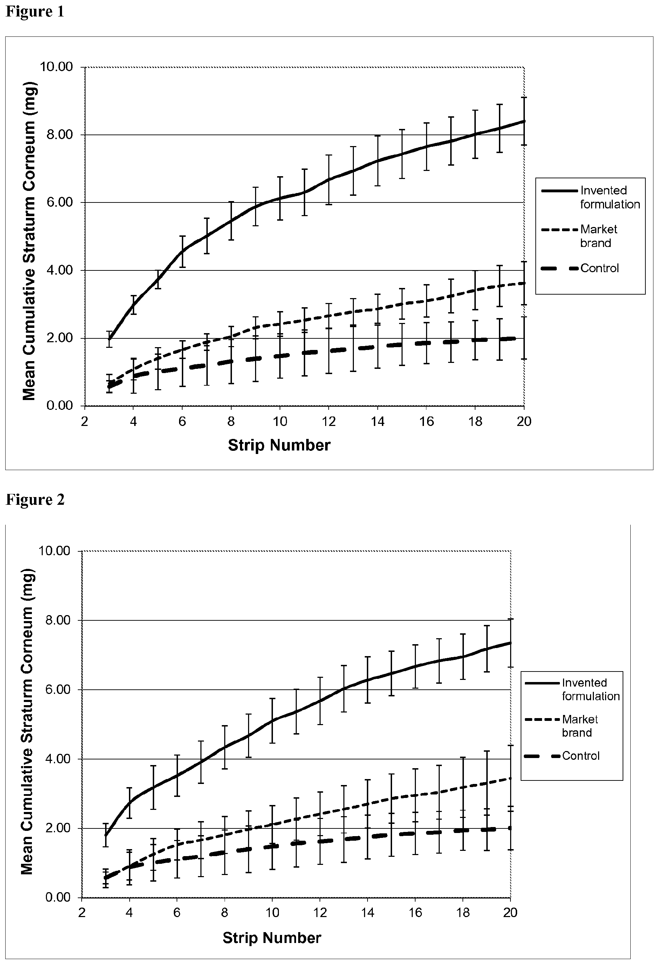

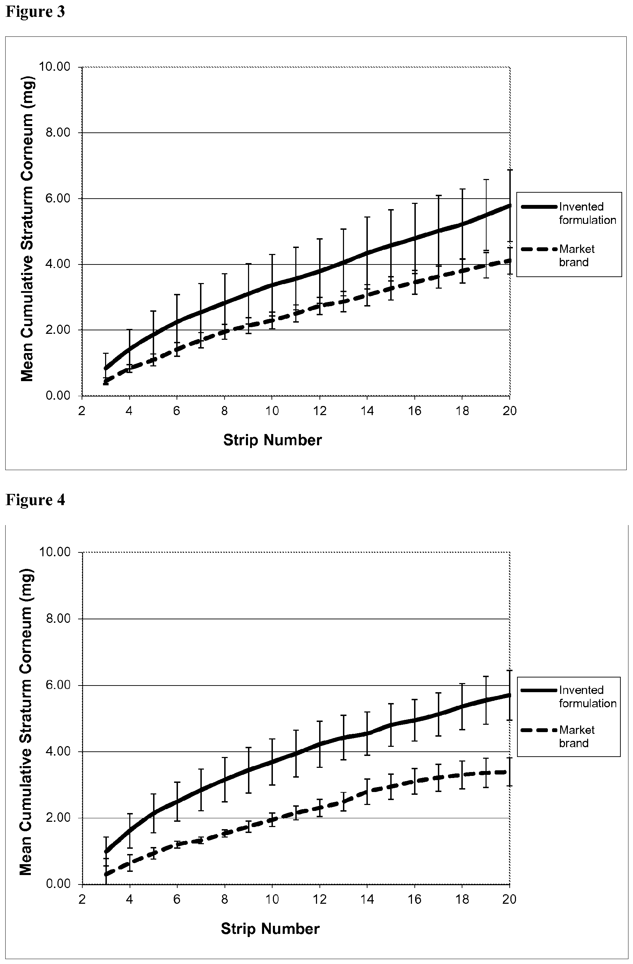

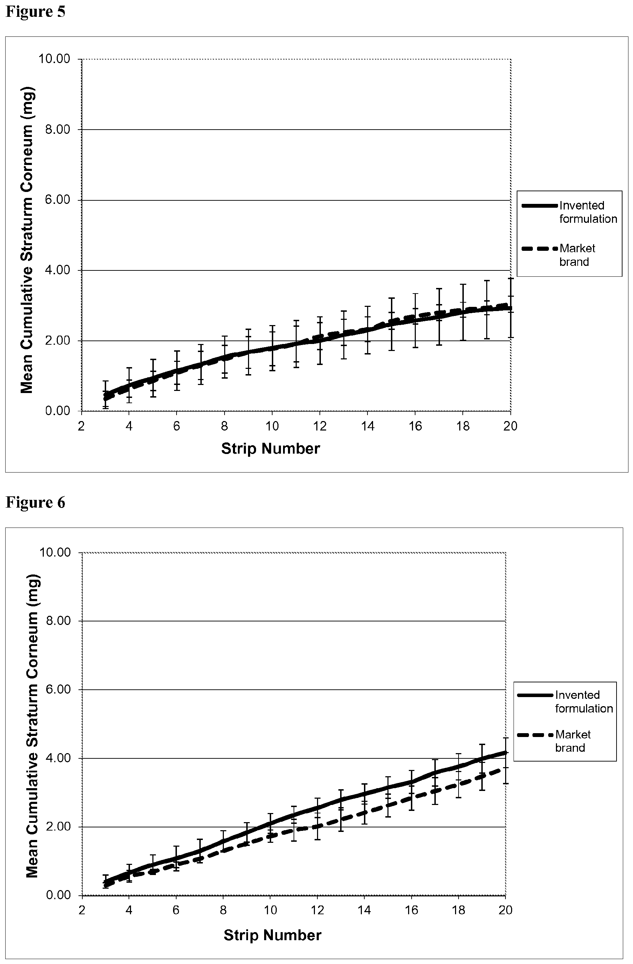

The results of the disc weighings are shown in Figures 1 to 6. Each of these graphs plots the mean cumulative weight of stratum corneum (SC) removed for each of the 20 tape strips, for the formulation according to the invention, the commercially available product ("market brand") and the control. Weight of SC removed is assumed to correlate with keratolytic (SC cell loosening) activity. Figures 1 to 6 relate to samples taken 0.2 hours, 0.5 hours, 1 hour, 2 hours, 4 hours and 24 hours respectively after the application of the test formulations.

These data show that the keratolytic effect of the formulation according to the invention is faster in onset, and more pronounced, than that of the market brand. Enhanced keratolytic activity can be observed within minutes of treatment. Moreover, the activity persists for several hours after the formulation has been applied to the skin, and indeed is noticeable even 24 hours after treatment.

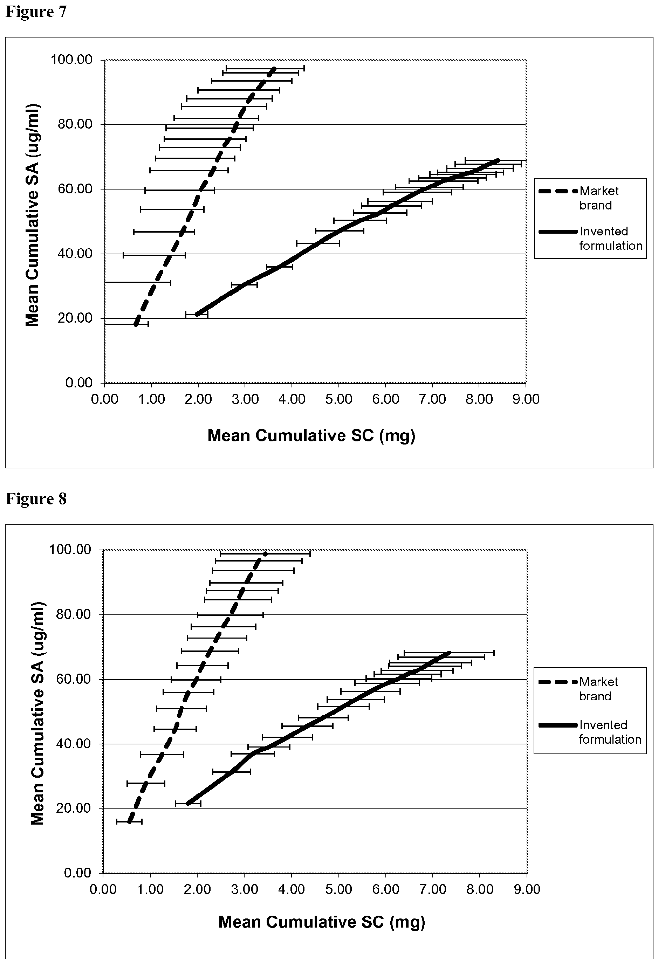

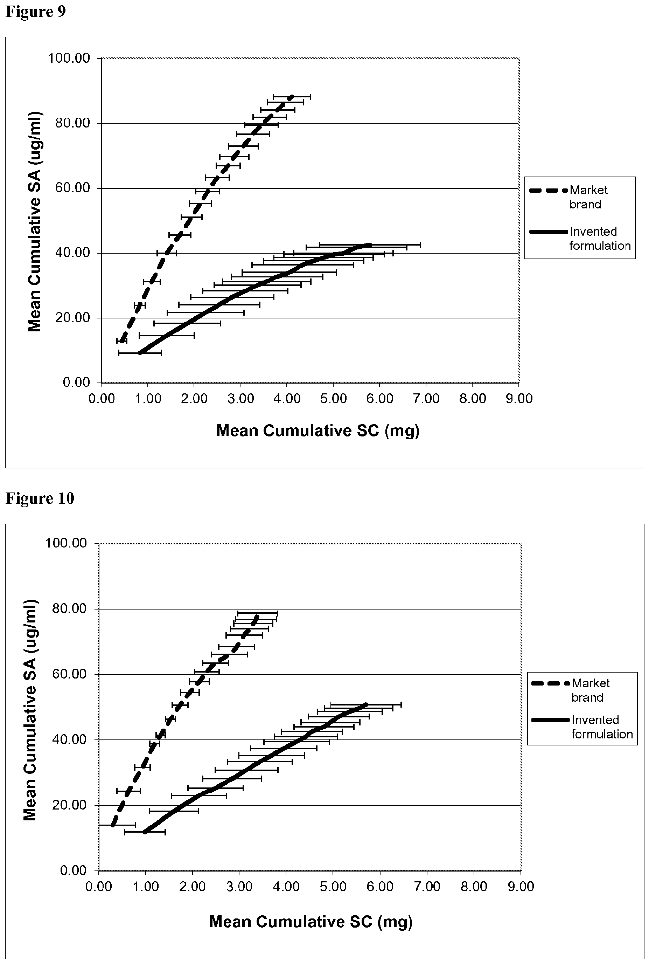

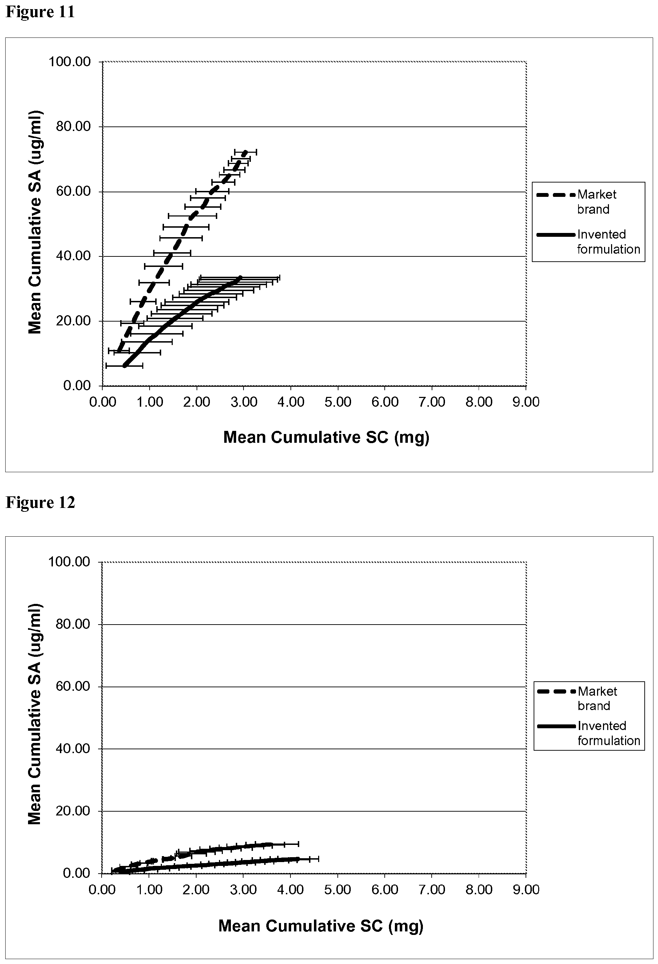

The results of the HPLC analyses are shown in Figures 7 to 12. Each of these graphs plots mean cumulative SA quantities against mean cumulative SC quantities (equivalent to SC depth). Figures 7 to 12 relate, respectively, to samples taken 0.2 hours, 0.5 hours, 1 hour, 2 hours, 4 hours and 24 hours after the application of the test formulations.

It can be seen that the formulation of the invention results in the SA permeating to a greater depth in the stratum corneum, as compared to the commercially available product. The SA also permeates faster from the invented formulation. The recovery profiles support the theory that the invented formulation results in greater SC penetration of the SA active, at all sampling timepoints. Twice as much SA has penetrated the SC one hour after treatment with the invented formulation, as compared to the market brand, and SA activity appears to occur at a much deeper level within the SC.

The improved keratolytic efficacy of a formulation according to the invention is likely to be of value in the topical treatment of acne. Loosening and removal of stratum corneum cells can help to unblock skin pores, and in turn to reduce the severity of both inflamed and non-inflamed acne lesions.

The invented formulation may for instance be used either to treat, or to help prevent, acne, or another skin or skin structure condition which is amenable to treatment using a keratolytic agent (for example dandruff, psoriasis, calluses, corns, keratosis pilaris or warts). It may be administered topically to the skin - for example to acne-affected skin -, in particular as a "leave-on" treatment, and can provide sustained efficacy of the salicylic acid or derivative for several hours after application. The formulation can therefore be used as part of a daily (and/or nightly) skin treatment regime.

### Example 3 - formulations B to E

Further skin treatment formulations B to E were prepared using the components and concentrations listed in Table 2. Concentrations are quoted as percentages by weight (% w/w). Formulations B and C, which contained no thickener, had the form of solutions rather than gels.

**Table 2**

| ***Ingredient*** | ***B*** | ***C*** | ***D*** | ***E*** |
|---|---|---|---|---|
| Copper usnate | 1 | 1 | 1 | 1 |
| DMI | 30 | 30 | 30 | 29.95 |
| Homosalate | 10 | 10 | 10 | 9.98 |
| Glyceryl diisostearate | 20 | 38 | | |
| PCA glyceryl oleate | | | 5 | 4.99 |
| THFA | 20 | | | |
| Ethanol | | 19 | | |
| PEG-8 caprylic/capric glycerides | 18 | | 10 | 10 |
| Ethyl pyrrolidone | | | 20 | 19.97 |
| Methyl glucose dioleate | | | 5 | 4.99 |
| Ethyl pyruvate | | | 14.5 | 14.55 |
| Oleic acid | | | 0.5 | 0.57 |
| PCA | | 1 | | |
| Antioxidant | | | 1 | 1 |
| Thickener | | | 2 | 2 |
| Salicylic acid | 1 | 1 | 1 | 1 |
| Total | 100 | 100 | 100 | 100 |

The THFA and ethanol were sourced from Sigma-Aldrich, UK, and the glyceryl diisostearate from Dr Straetmans Chemische Produkte GmbH.

The formulations were prepared using an analogous method to that of Example 1. The copper usnate was premixed with the DMI, the homosalate and where appropriate the ethyl pyrrolidone, THFA and ethyl pyruvate. The mixture was heated and stirred to optimise usnate dissolution, then filtered to remove any undissolved residue. The glyceryl fatty acid ester was then added, along with the remaining components of the solvent system and the oleic acid where necessary, and mixed vigorously. To this mixture was added, as appropriate, the PCA, salicylic acid and antioxidant, followed by the thickener. The resultant mixture was stirred and heated, shaken vigorously and if necessary left to gel at room temperature.

### Example 4 - antimicrobial activity in vivo (formulations B to E)

These experiments used formulations B to E as described in Example 3, containing salicylic acid as a keratolytic agent and copper usnate as an antimicrobial agent. The effects of the formulations on the skin surface populations of coagulase-negative staphylococci and propionibacteria were tested on human volunteers.

Each test formulation was topically applied, twice daily, to three locations on the face of a healthy human volunteer: the forehead (FH), left cheek (LC) and right cheek (RC). The skin was sampled before the treatment began, and at intervals afterwards whilst the treatment continued, using the method of Williamson-Kligman (Williamson P and Kligman AM, "A New Method for the Quantitative Investigation of Cutaneous Bacteria", J Invest Dermatol 1965 Dec; 45(6): 498-503). Samples were collected in the mornings, in each case between 10 and 12 hours after the previously applied evening dose and before application of the morning dose. Samples were also collected two weeks after the last topical application of the formulation.

The samples were assessed for numbers of coagulase-negative staphylococci and *Propionibacterium* spp by serial dilution and viable counting on suitable media (for the staphylococci, Columbia blood agar (Oxoid) containing 5% defibrinated horse blood (E & O Laboratories), and for the propionibacteria, tryptone (Oxoid, 2%), yeast extract (Oxoid, 1%), glucose (Sigma, 0.5%) agar containing 2 mg/L of furazolidone (Sigma). Colony counts, obtained following incubation at 37°C aerobically for 24 hours in the case of staphylococci and anaerobically for 7 days in the case of propionibacteria, were used to calculate the log number of bacteria per cm² of skin.

A decline in the numbers of bacteria present at the skin surface, over the course of the treatment period, would indicate that bacteria were being successfully inactivated not only at the skin surface but also in the follicles.

The results are shown in Tables 3a to 3d below, for formulations B to E respectively.

**Table 3a (formulation B)**

| ***Days of application*** | ***Coagulase-negative staphylococci log count*/*cm² skin*** | | | | ***Change from baseline*** |
|---|---|---|---|---|---|
| | **FH** | **LC** | **RC** | **Mean** | |
| 0 (baseline) | 2.67 | 4.09 | 4.80 | 3.85 | n/a |
| 7 | 2.26 | 2.40 | 2.31 | 2.32 | -1.53 |

| ***Days of application*** | ***Propionibacteria log count*/*cm² skin*** | | | | ***Change from baseline*** |
|---|---|---|---|---|---|
| | **FH** | **LC** | **RC** | **Mean** | |
| 0 (baseline) | 5.53 | 5.61 | 6.28 | 5.81 | n/a |
| 7 | 4.78 | 5.09 | 3.52 | 4.46 | -1.35 |

**Table 3b (formulation C)**

| ***Days of application*** | ***Coagulase-negative staphylococci log count*/*cm² skin*** | | | | ***Change from baseline*** |
|---|---|---|---|---|---|
| | **FH** | **LC** | **RC** | **Mean** | |
| 0 (baseline) | 5.68 | 4.04 | 4.67 | 4.9 | n/a |
| | 5.73 | 4.40 | 4.75 | | |
| 5 | 2.31 | 3.13 | 3.12 | 2.85 | -2.05 |

| ***Days of application*** | ***Propionibacteria log count*/*cm² skin*** | | | | ***Change from baseline*** |
|---|---|---|---|---|---|
| | **FH** | **LC** | **RC** | **Mean** | |
| 0 (baseline) | 6.62 | 6.18 | 6.34 | 6.38 | n/a |
| | 6.56 | 6.12 | 6.43 | | |
| 5 | 5.77 | 5.75 | 6.15 | 5.89 | -0.48 |

**Table 3c (formulation D)**

| ***Days of application*** | ***Coagulase-negative staphylococci log count*/*cm² skin*** | | | | ***Change from baseline*** |
|---|---|---|---|---|---|
| | **FH** | **LC** | **RC** | **Mean** | |
| 0 (baseline) | 5.10 | 5.14 | 4.24 | 4.76 | n/a |
| | 5.20 | 4.64 | 4.22 | | |
| 3 | 1.61 | 3.54 | 1.99 | 2.38 | -2.38 |
| 7 | 2.02 | 2.33 | 1.79 | 2.05 | -2.71 |
| 10 | 1.21 | 2.29 | 2.14 | 1.88 | -2.88 |
| 11 | 1.21 | 2.18 | 2.93 | 2.11 | -2.65 |
| 14 | 2.33 | 2.53 | 3.21 | 2.69 | -2.07 |

| ***Days of application*** | ***Propionibacteria log count*/*cm² skin*** | | | | ***Change from baseline*** |
|---|---|---|---|---|---|
| | **FH** | **LC** | **RC** | **Mean** | |
| 0 (baseline) | 6.19 | 6.33 | 6.70 | 6.45 | n/a |
| | 6.53 | 6.43 | 6.49 | | |
| 3 | 4.33 | 5.75 | 5.46 | 5.18 | -1.27 |
| 7 | 4.97 | 5.65 | 5.48 | 5.37 | -1.08 |
| 10 | 3.86 | 5.50 | 5.05 | 4.80 | -1.64 |
| 11 | 4.66 | 5.53 | 4.97 | 5.05 | -1.39 |
| 14 | 3.93 | 5.58 | 5.33 | 4.95 | -1.50 |

**Table 3d (formulation E)**

| ***Days of application*** | ***Coagulase-negative staphylococci log count*/*cm² skin*** | | | | ***Change from baseline*** |
|---|---|---|---|---|---|
| | **FH** | **LC** | **RC** | **Mean** | |
| 0 | 1.51 | 4.46 | 4.15 | 3.37 | n/a |
| 7 | 0.91 | 2.34 | 1.89 | 1.71 | -1.66 |
| 12 | 2.55 | 2.72 | 2.71 | 2.66 | -0.71 |
| 20 | 1.31 | 2.21 | 2.68 | 2.07 | -1.31 |
| ***Days of application*** | ***Propionibacteria log count*/*cm² skin*** | | | | ***Change from baseline*** |
| | **FH** | **LC** | **RC** | **Mean** | |
| 0 | 5.09 | 6.13 | 6.12 | 5.78 | n/a |
| 7 | 2.23 | 5.66 | 3.37 | 3.75 | -2.03 |
| 12 | 2.49 | 5.34 | 4.57 | 4.13 | -1.65 |
| 20 | 2.21 | 5.81 | 5.78 | 4.60 | -1.18 |

In all cases, the invented formulations caused a marked reduction in numbers of both coagulase-negative staphylococci and propionibacteria compared to the pre-treatment values. These data demonstrate that a formulation containing both salicylic acid and an usnate salt solubilised in the components (i) to (iii) can effectively deliver the usnate to the skin, including to bacteria present in the follicles. Such a formulation is therefore suitable for use as a topical antimicrobial agent, and as a topical anti-acne treatment.

It can be seen from these data that a combined salicylic acid and usnate formulation according to the invention can be effective in vivo as an antimicrobial agent against coagulase-negative staphylococci. It can also be effective in vivo against propionibacteria. It appears to be reaching the targeted follicles and once there, to be successfully acting against the resident bacteria, significantly reducing the numbers of bacteria present at the skin surface following treatment. The formulation is therefore suitable for use as a topical antimicrobial agent. In particular, it can be of use as a topical anti-acne preparation, in which the two modes of action (the keratolytic effect of the salicylic acid combined with the antibacterial activity of the usnate) can complement one another to provide an overall more effective treatment.

Moreover, this and Example 2 show that both the antibacterial and the keratolytic effects appear to be sustained for a significant period of time following each topical application, making the formulation suitable for use as a "leave-on" skin treatment product. The data demonstrate the substantivity of the formulation, suggesting that the salicylic acid and the usnate remain in the stratum corneum, where they can continue to exert their biological effects, for some time after application to the skin. This would be consistent with delivery of the actives to the follicles via the stratum corneum.

### Example 5 - antimicrobial activity in vivo (formulation D)

Six human volunteers were treated for 14 days using formulation D from Example 3, using similar treatment regimens and sampling techniques as described in Example 4. Surface propionibacteria and staphylococci were sampled using the scrub method of Williamson and Kligman, again as described in Example 4. Follicular propionibacteria and staphylococci were also enumerated using the cyanoacrylate glue technique (Holland KT, Roberts CD 1974, "A technique for sampling micro-organisms from the pilo-sebaceous ducts", J Appl Bacteriol 37(3): 289-96).

The results are shown in Tables 4 and 5 below, for the surface and follicular bacteria respectively.

**Table 4**

| ***Days of application*** | ****Coagulase-negative staphylococci Mean log count*/*cm² skin*** | ***Change from baseline*** | *****P value*** |
|---|---|---|---|
| 0 (baseline) | 3.9 | n/a | n/a |
| 1 | 2.9 | -1.0 | <0.0001 |
| 4 | 2.3 | -1.6 | <0.0001 |
| 7 | 2.5 | -1.4 | <0.0001 |
| 10 | 2.2 | -1.7 | <0.0001 |
| 14 | 2.4 | -1.5 | <0.0001 |

| ***Days of application*** | ***Propionibacteria Mean log count*/*cm² skin*** | ***Change from baseline*** | *****P value*** |
|---|---|---|---|
| 0 (baseline) | 5.6 | n/a | n/a |
| 1 | 4.9 | -0.7 | 0.0002 |
| 4 | 4.5 | -1.1 | <0.0001 |
| 7 | 4.5 | -1.1 | <0.0001 |
| 10 | 4.5 | -1.1 | <0.0001 |
| 14 | 4.6 | -1.0 | 0.0007 |

| | | | |
|---|---|---|---|
| * Mean values were calculated from the data obtained from six volunteers sampled at three sites: forehead, right cheek and left cheek. ** A statistical analysis was carried out using a paired Student's t test. Significance is indicated by P ≤ 0.05. | | | |

**Table 5**

| ***Days of application*** | ****Coagulase-negative staphylococci Mean log count*/*cm² skin*** | ***Change from baseline*** | *****P value*** |
|---|---|---|---|
| 0 (baseline) | 3.7 | n/a | n/a |
| 14 | 0.5 | -3.2 | 0.001 |

| ***Days of application*** | ***Propionibacteria Mean log count*/*cm² skin*** | ***Change from baseline*** | *****P value*** |
|---|---|---|---|
| 0 (baseline) | 5.3 | n/a | n/a |
| 14 | 3.9 | -1.4 | 0.1 |

| | | | |
|---|---|---|---|
| * Mean values were calculated from the data obtained from six volunteers sampled at three sites from the central forehead. ** A statistical analysis was carried out using a paired Student's t test. Significance is indicated by P ≤ 0.05. | | | |

Table 4 shows that formulation D significantly reduced the numbers of coagulase-negative staphylococci and propionibacteria at the skin surface of this treatment cohort. Table 5 shows that the formulation also produced a highly significant reduction in the follicular staphylococci and a mean log cm⁻² reduction in follicular propionibacteria of 1.4. Thus, again, the formulation has been demonstrated to be capable of exerting antimicrobial activity within the pilosebaceous follicles.

This further confirms that a formulation according to the invention can represent a powerful combination of a highly effective keratolytic and a potent antimicrobial agent, both of which can be delivered efficiently and with lasting effect to the target areas of the skin. Such a formulation can be particularly beneficial for the treatment of inflammatory lesions (Waller et al, 2006, Skin Physiol, 19: 283-289). It can also help to target the most problematic spots, providing a triple action against bacterial infection, redness and blocked pores.

### Example 6 - in vivo trial (formulation D)

Formulation D, as described in Example 3, was tested on thirteen human volunteers. The volunteers were selected to have between 15 and 60 inflamed spots on the face and to be aged between 15 and 35 years. Each was treated for 6 weeks by applying formulation D to the entire face (as a leave-on treatment) twice daily.

Examinations took place at weeks 0, 2, 4 and 6 with facial spot counts being carried out at the visits by a trained assessor according to the Leeds revised system (Burke BM & Cunliffe WJ, "The assessment of acne vulgaris: The Leeds technique", Br J Dermatol, 1984, 111: 83-92). Inflamed spot (IS), non-inflamed spot and total spot counts were recorded for each participant over the full six weeks.

A decline in the number of inflamed spots compared to baseline counts would indicate clinical efficacy and suggest the formulation was a good candidate for the management of spot prone skin.

The results of the trial, for inflamed spot counts, are shown in Table 6 below.

**Table 6**

| | ***Weeks of application*** | | | |
|---|---|---|---|---|
| | ***T*=*0*** | ***T*=*2*** | ***T*=*4*** | ***T*=*6*** |
| Mean IS count * | 39.2 | 29.3 | 26.7 | 26.6 |
| % Mean change | - | -25.3 | -31.9 | -32.1 |
| P values ** | - | 0.351 | 0.041 | 0.126 |

| | | | | |
|---|---|---|---|---|
| * Mean values were calculated from the data obtained from twelve volunteers. ** A statistical analysis was carried out using a paired Student's t test. Significance is indicated by P ≤ 0.05. | | | | |

The Table 6 data show a significant reduction in inflamed spots (31.9%) after 4 weeks. Inflamed spots were reduced throughout the study, although the results appeared not to be significant at 2 and 6 weeks.

This trial indicates that a formulation according to the invention can be a viable anti-acne treatment, with activity comparable to currently marketed products.

### Example 7 - formulations F to H

Further skin treatment formulations F to H were prepared, containing higher salicylic acid concentrations. Table 7 below lists the components and concentrations used (concentrations are quoted as percentages by weight). Again the solvent system of the invention was shown capable of completely solubilising the salicylic acid and copper usnate actives.

**Table 7**

| ***Ingredient*** | ***F*** | ***G*** | ***H*** |
|---|---|---|---|
| Copper usnate | 1 | 1 | 1 |
| DMI | 30 | 30 | 30 |
| Homosalate | 10 | 10 | 10 |
| Glyceryl diisostearate | 20 | 20 | 20 |
| THFA | 20 | 20 | 10 |
| PEG-8 caprylic/capric glycerides | 14 | 9 | 9 |
| Salicylic acid | 5 | 10 | 20 |
| Total | 100 | 100 | 100 |

The components of these formulations were sourced in the same way as for Examples 1 and 3. The formulations were prepared in the following way. The copper usnate was premixed with the DMI, homosalate, THFA, salicylic acid and PEG-8 caprylic/capric glycerides. This mixture was heated and stirred until the solid material had dissolved. The glyceryl fatty acid ester was then added, followed by vigorous mixing.

### Example 8 - formulations J to P

Further skin treatment gel formulations J to P were prepared using the components and concentrations listed in Table 8. Concentrations are quoted as percentages by weight. Formulations N and P corresponded to J and K respectively, but contained no usnic acid or usnate co-active.

**Table 8**

| ***Ingredient*** | ***J*** | ***K*** | ***L*** | ***M*** | ***N*** | ***P*** |
|---|---|---|---|---|---|---|
| Salicylic acid | 1.00 | 1.00 | 1.00 | 1.00 | 1.00 | 1.00 |
| DMI | 30.00 | 30.00 | 30.00 | 30.00 | 30.00 | 30.00 |
| Homosalate | 8.50 | 8.50 | 8.50 | 7.00 | 8.50 | 8.50 |
| Glyceryl diisostearate | | 5.00 | 5.00 | 5.00 | | 5.00 |
| PCA glyceryl oleate | 5.00 | | 5.00 | | 5.00 | |
| THFA | 10.00 | 15.00 | 10.00 | 15.00 | 10.00 | 15.00 |
| Ethanol | 10.00 | 5.00 | 10.00 | 5.00 | 10.00 | 5.00 |
| Labrasol™ | 20.00 | 25.00 | 15.00 | 22.00 | 21.00 | 26.00 |
| Methyl glucose dioleate | 5.00 | 5.00 | 5.00 | 5.00 | 5.00 | 5.00 |
| Hydroxypropyl cellulose (GF Pharm) | 2.50 | 2.50 | 2.50 | | 2.50 | 2.50 |
| Carbopol™ Ultrez 10 | | | | 7.00 | | |
| Copper usnate | 1.00 | 1.00 | 1.00 | 1.00 | | |
| PEG 400 | 7.00 | 2.00 | 7.00 | 2.00 | 7.00 | 2.00 |
| Total | 100.00 | 100.00 | 100.00 | 100.00 | 100.00 | 100.00 |

The DMI was sourced as Arlasolve DMI from Croda, the THFA from Pennakem, the Labrasol™ from Gattefosse, the homosalate from Symrise, the methyl glucose dioleate from Lubrizol, the PCA glyceryl oleate as DermoFeel™ P-30 from Dr Straetmans Chemische Produkte GmbH, the glyceryl diisostearate from Stearinierer Dubois, the salicylic acid(BP grade) from A & E Cannock, the hydroxypropyl cellulose thickener as GF Pharm (molecular weight 370,000) from Ashland, the Carbopol™ gelling agent from Lubrizol, the ethanol from Merck or Fisher Scientific and the PEG 400 from Merck. The copper usnate was sourced from Onyx Scientific Ltd, UK.

Formulations J to P were prepared by loading the copper usnate (where applicable) into a clean, dry vessel, and then adding, stepwise, the ingredients (a) DMI, (b) homosalate, (c) THFA, (d) ethanol, (e) PEG 400, (f) Labrasol™ and (g) salicylic acid. The resultant mixture was protected from light using aluminium foil, placed in a water bath at 50°C and stirred until the copper usnate was fully dissolved. The PCA glyceryl oleate or glyceryl diisostearate was then added, and also the methyl glucose dioleate, and again this mixture was protected from light and stirred in a water bath at 50°C.

The mixture was then removed from the water bath and cooled to ambient temperature. The thickener (hydroxypropyl cellulose or Carbopol™) was added gradually, whilst homogenising the mixture, until evenly dispersed. The resultant formulation was then stirred at room temperature for at least 16 hours to ensure solvation of the copper usnate and/or salicylic acid.

These gel formulations were found to have an acceptable appearance and odour, an even consistency and a pleasing, non-greasy, feel when applied to the skin. They were also absorbed fairly quickly into the skin and were easy to apply. Formulation K performed particularly well in terms of skin feel, formulation J in terms of odour. In all cases, the salicylic acid active was completely dissolved in the solvent system at 1% w/w, as was the additional active copper usnate in formulations J to M.

Examples 9 to 11 below also demonstrate that the formulations have good physical and chemical stability.

### Example 9 - stability tests i (formulations J. K, N and P)

Samples of formulations J, K, N and P were stored at 40°C and 75% RH for 12 weeks. At certain time points, the samples were assessed for changes in their viscosity, using a Brookfield™ LV-DV-1+ viscometer with Spindle 25 at 12 rpm. The viscosities of formulations K and P were also assessed using Spindle 34 at 6 rpm, due to a low torque value with Spindle 25 All viscosities were determined at 25°C.

The results are shown in Table 9 (appended). It can be seen that formulation J and its usnate-free equivalent N showed no notable change in viscosity during the storage period. Formulations K and P performed marginally less well, exhibiting a small reduction in viscosity and also demonstrating a slight inconsistency in texture during the tests.

### Example 10 - stability tests ii (formulations J. K, N and P)

Samples of formulations J, K, N and P were stored at 40°C and 75% RH for 12 weeks. At certain time points, the samples were assessed for changes in their macroscopic and microscopic appearance. Macroscopic appearance was judged by eye, primarily to detect changes in physical stability and phase separation. Colour was assessed using the Pantone Formula Guide. Microscopic observations were made by light microscopy, with magnifications of 200x and 400x, and using both polarised and non-polarised light, in order to detect the emergence of precipitated copper usnate crystals.

The results are summarised in appended Table 10. During the storage period there were no noticeable differences in the colours of any of the four formulations. In the formulations containing copper usnate as a co-active, there was no microscopic evidence of copper usnate crystals. Formulations J and N performed particularly well, showing no evidence of changes in their physical stability over the full 12 week stability period. In formulations K and P, there was some increase in agglomeration during storage. It is possible that this might be due to the higher concentration of THFA in formulations K and P, in combination with the other excipients present, leading to less consistent gelling properties, although we do not wish to be bound by this theory.

### Example 11 - stability tests iii (formulations J, K, N and P)

The formulation samples used in Examples 9 and 10 were stored in 15 mL polypropylene airless pump dispensers, such as might typically be used to store skin treatment formulations. These pumps were assessed visually, and also weighed in order to detect potential weight loss, at intervals during the 12 week stability test period. No noticeable weight changes were observed, and no changes in the internal appearance of the pumps when in contact with any of the test formulations.

Thus, the results from Examples 9 to 11 indicate the stability of formulations according to the invention, their suitability as vehicles for the keratolytic agent salicylic acid, and their potential use for the topical treatment of the skin.

### Example 12 - skin patch tests (formulations J and K)

Samples of each of the formulations J and K were applied to the skin of three human volunteers using 8mm Finn chamber patch test units. After 24 hours the test strips were carefully removed and the results read. Each patch test was scored according to the reaction seen on the skin, using the grading system of Wilkinson et al (Wilkinson DS, Fregert S, Magnusson B, Bandmann HJ, Calnan CD, Cronin E, Hjorth N, Maibach HJ, Malalten KE, Meneghini CL, Pirilä V, "Terminology of contact dermatitis", Acta Derm Venereol (1970), 50(4): 287-92). This grading system is summarised in Table 11 below.

**Table 11**

| ***Score*** | ***Interpretation*** |
|---|---|
| - | Negative reaction |
| ?+ | Doubtful reaction, faint erythema only |
| + | Weak (nonvesicular) reaction, erythema, slight infiltration |
| ++ | Strong (edematous or vesicular) reaction; erythema, infiltration, vesicles |
| +++ | Extreme reaction (bullous and ulcerative) |
| IR | Irritant reactions of different types |
| NT | Not tested |

Both test formulations produced a negative reaction in all three of the volunteers, ie no indication of skin irritancy. This confirms their suitability for use as topical skin treatment formulations.

### Example 13 - sensory evaluation (formulations J and K)

Seven human volunteers were asked to apply small amounts of a test formulation to a section of their forearm. They were asked to rub in the formulation as they would any other cosmetic product, noting how the formulation looked, felt and behaved on and after application. The volunteers were then asked to complete a questionnaire relating to the sensory profile of the formulation. This process was repeated until each volunteer had evaluated seven test formulations, which included both formulations J and K and also a commercially available topical spot treatment cream. The different formulations were randomly assigned for application to different sections of the forearm for each volunteer, and the volunteers did not know the identities or contents of the formulations they were applying.

When asked initially to rate the appearance of the formulations, none of the volunteers expressed dislike for formulations J and K. 14.3% of the volunteers liked both the formulations a lot. As regards the colour of the formulations, none of the volunteers expressed dislike for formulation J, although 14.3% did express a slight dislike for the colour of formulation K. As regards the consistency of the formulations, 100% of the volunteers assessed formulation K as being "about right" (ie neither too thick nor too thin), and 71.4% assessed formulation J as being "about right".

The volunteers were then asked how pleasant or unpleasant the formulation felt on application to the skin. 100% of them regarded formulation J as either "very pleasant", "fairly pleasant" or "neither pleasant nor unpleasant": 85.8% categorised formulation K in this way. As regards the speed with which the formulations were perceived to absorb into the skin, 57.2% of the volunteers categorised formulation J as absorbing "very quickly", "fairly quickly" or "neither quickly nor slowly", whilst 100% categorised formulation K in this way (85.7% believing that formulation K was absorbed "fairly quickly").

None of the volunteers categorised either of the formulations J and K as being difficult to spread on the skin: in fact, 100% of them regarded formulation J as being either "very easy" or "fairly easy" to spread, whilst 85.8% of them categorised formulation K in this way and the remainder regarded formulation K as "neither easy nor difficult" to spread.

After application of the test formulation to the skin, 57.1% of the volunteers believed that formulation K left their skin feeling "neither greasy nor dry" (as compared to 42.9% feeling the same way about the commercially available product). For formulation J, 85.8% of the volunteers said that their skin felt either "fairly greasy", "fairly dry" or "neither greasy nor dry".

These results indicate that the formulations according to the invention are generally acceptable in terms of their sensory performance, for application to human skin. Not only are they chemically and physically stable (as demonstrated for instance in Examples 9 to 11), and extremely effective delivery vehicles for the keratolytic agent salicylic acid (Examples 2 and 6), but they also look and feel appropriate to consumers and apply well to the skin, without (Example 12) causing irritation.

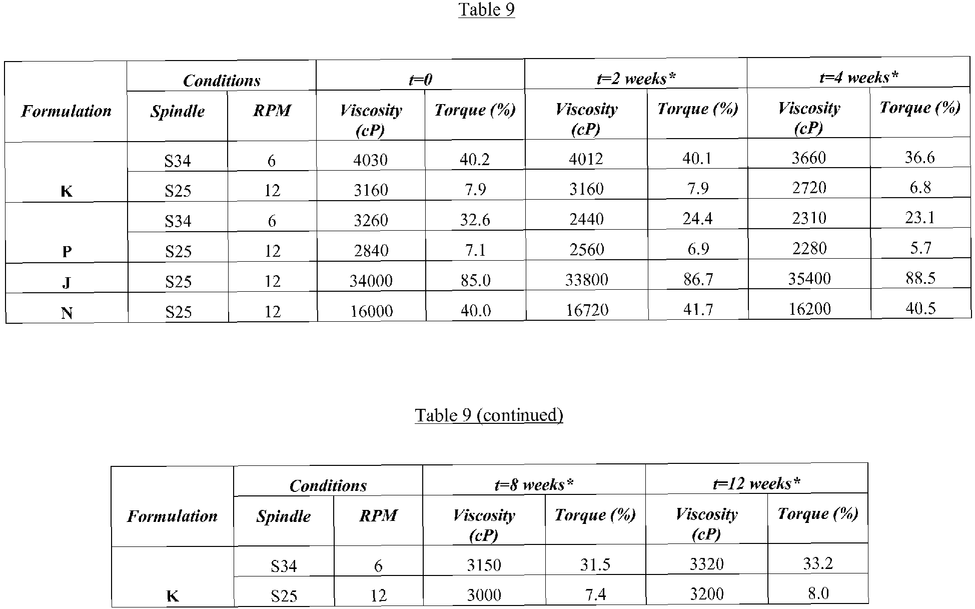

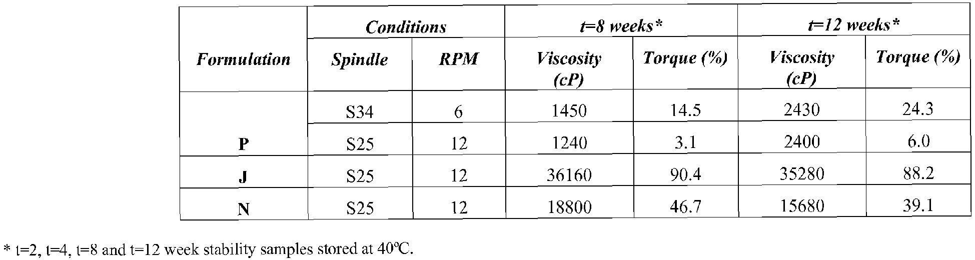

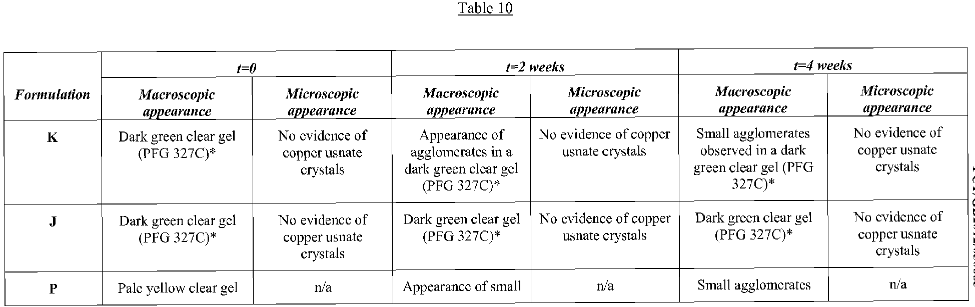

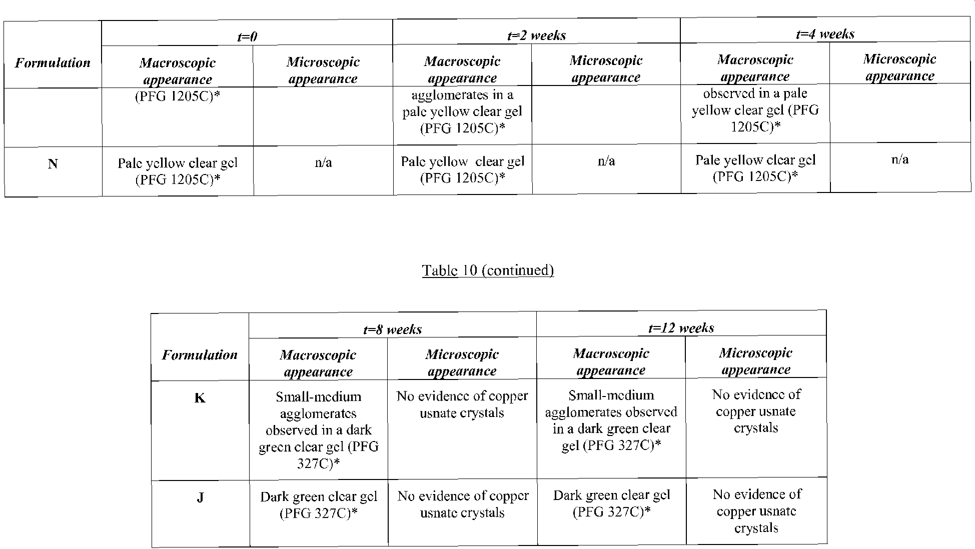

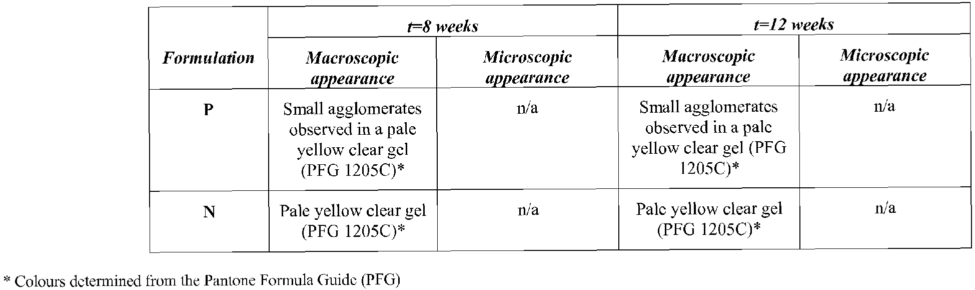

## Claims

1. A topical skin treatment formulation containing salicylic acid dissolved in a solvent system, wherein the solvent system comprises:
(i) dimethyl isosorbide (DMI);
(ii) a C1 to C9 alkyl salicylate; and
(iii) a glyceryl fatty acid ester.

2. A formulation according to claim 1, wherein the solvent system additionally comprises (iv) an alcohol.

3. A formulation according to claim 1 or claim 2, wherein the solvent system additionally comprises (v) a polyoxyalkylene-based solvent selected from polyoxyalkylene glyceryl esters, polyalkylene glycols and mixtures thereof.

4. A formulation according to any one of the preceding claims, wherein the solvent system additionally comprises a C1 to C4 alkyl glucose ester.

5. A formulation according to any one of the preceding claims, wherein the solvent system comprises:
(1) DMI, at a concentration from 25 to 35% w/w;
(2) homosalate, at a concentration from 5 to 12% w/w;
(3) a glyceryl fatty acid ester selected from PCA glyceryl oleate, glyceryl diisostearate and mixtures thereof, at a concentration from 2 to 8% w/w;
(4) an alcohol selected from ethanol, THFA and mixtures thereof, at a concentration from 15 to 25% w/w;
(5) a component selected from polyoxyethylene glyceryl esters, PEGs, and mixtures thereof, at a concentration from 22 to 32% w/w; and
(6) a methyl glucose ester, at a concentration from 2 to 8% w/w.

6. A formulation according to any one of the preceding claims, wherein the concentration of the salicylic acid in the formulation is from 0.5 to 1.5% w/w.

7. A formulation according to any one of the preceding claims, which contains one or more thickening agents.

8. A formulation according to any one of the preceding claims, which is in the form of a solution, lotion, cream or gel.

9. A formulation according to any one of the preceding claims, which additionally comprises usnic acid or an usnate salt.

10. A product which incorporates a formulation according to any one of the preceding claims.

11. A formulation according to any one of claims 1 to 9, for use in treatment carried out on a living human or animal, in particular human, body.

12. A formulation according to any one of claims 1 to 9, for use according to claim 11, wherein the condition is acne.

13. Formulation according to any one of claims 1 to 9, for use in the treatment of a condition (in particular a skin or skin structure condition) which is amenable to treatment by a keratolytic agent, or of a condition which is caused by, transmitted by and/or exacerbated by microbial activity.

14. Formulation according to any one of claims 1 to 9, and/or of a solvent system as defined in any one of claims 1 to 9, for use in improving the delivery of salicylic acid to a target site in or on the skin.

## Patentansprüche

1. Formulierung zur topischen Hautbehandlung, die in einem Lösemittelsystem gelöste Salicylsäure enthält, wobei das Lösemittelsystem umfasst:
(i) Dimethylisosorbid (DMI),
(ii) ein C1- bis C9-Alkylsalicylat und
(iii) einen Glycerylfettsäureester.

2. Formulierung nach Anspruch 1, wobei das Lösemittelsystem zusätzlich (iv) einen Alkohol umfasst.

3. Formulierung nach Anspruch 1 oder Anspruch 2, wobei das Lösemittelsystem zusätzlich (v) ein Polyoxyalkylen-basiertes Lösemittel umfasst, das ausgewählt ist aus Polyoxyalkylenglycerylestern, Polyalkylenglykolen und Mischungen davon.

4. Formulierung nach einem der vorhergehenden Ansprüche, wobei das Lösemittelsystem zusätzlich einen C1- bis C4-Alkylglukoseester umfasst.

5. Formulierung nach einem der vorhergehenden Ansprüche, wobei das Lösemittelsystem umfasst:
(1) DMI in einer Konzentration von 25 bis 35 Gew.-%,
(2) Homosalat in einer Konzentration von 5 bis 12 Gew.-%,
(3) einen Glycerylfettsäureester, der ausgewählt ist aus PCA-Glyceryloleat, Glyceryldiisostearat und Mischungen davon, in einer Konzentration von 2 bis 8 Gew.-%,
(4) einen Alkohol, der ausgewählt ist aus Ethanol, THFA und Mischungen davon, in einer Konzentration von 15 bis 25 Gew.-%,
(5) einen Bestandteil, der ausgewählt ist aus Polyoxyethylenglycerylestern, PEGs und Mischungen davon, in einer Konzentration von 22 bis 32 Gew.-% und
(6) einen Methylglukoseester in einer Konzentration von 2 bis 8 Gew.-%.

6. Formulierung nach einem der vorhergehenden Ansprüche, wobei die Konzentration der Salicylsäure in der Formulierung 0,5 bis 1,5 Gew.-% beträgt.

7. Formulierung nach einem der vorhergehenden Ansprüche, die ein oder mehrere Verdickungsmittel enthält.

8. Formulierung nach einem der vorhergehenden Ansprüche, die in Form einer Lösung, Lotion, Creme oder eines Gels vorliegt.

9. Formulierung nach einem der vorhergehenden Ansprüche, die zusätzlich Usninsäure oder ein Usnatsalz umfasst.

10. Eine Formulierung nach einem der vorhergehenden Ansprüche beinhaltendes Produkt.

11. Formulierung nach einem der Ansprüche 1 bis 9 zur Verwendung bei einer an einem lebenden menschlichen oder tierischen, insbesondere menschlichen Körper durchgeführten Behandlung.

12. Formulierung nach einem der Ansprüche 1 bis 9 zur Verwendung nach Anspruch 11, wobei es sich bei der Erkrankung um Akne handelt.

13. Formulierung nach einem der Ansprüche 1 bis 9 zur Verwendung bei der Behandlung einer Erkrankung (insbesondere einer Erkrankung der Haut oder der Hautstruktur), die mit einem keratolytischen Wirkstoff behandelbar ist, oder einer Erkrankung, die durch mikrobielle Aktivität verursacht, übertragen und/oder verstärkt wird.

14. Formulierung nach einem der Ansprüche 1 bis 9 und/oder wie in einem der Ansprüche 1 bis 9 definiertes Lösemittelsystem zur Verwendung bei der Verbesserung der Abgabe von Salicylsäure an einen Zielort in oder auf der Haut.

## Revendications

1. Formulation pour le traitement topique de la peau contenant de l'acide salicylique dissous dans un système solvant, dans lequel le système solvant comprend :
(i) de l'isosorbide diméthylique (DMI) ;
(ii) un salicylate d'alkyle en C1 à C9 ; et
(iii) un ester d'acide gras de glycéryle.

2. Formulation selon la revendication 1, dans laquelle le système solvant comprend en outre (iv) un alcool.

3. Formulation selon la revendication 1 ou la revendication 2, dans laquelle le système solvant comprend en outre (v) un solvant à base de polyoxyalkylène sélectionné parmi les esters glycéryliques de polyoxyalkylène, les polyalkylène glycols et les mélanges de ceux-ci.

4. Formulation selon l'une quelconque des revendications précédentes, dans laquelle le système solvant comprend en outre un ester alkylique de glucose en C1 à C4.

5. Formulation selon l'une quelconque des revendications précédentes, dans laquelle le système solvant comprend :
(1) du DMI, à une concentration de 25 à 35 % p/p ;
(2) de l'homosalate, à une concentration de 5 à 12 % p/p ;
(3) un ester glycérylique d'acide gras sélectionné parmi le glycéryle oléate de PCA, le diisostéarate de glycéryle et les mélanges de ceux-ci, à une concentration de 2 à 8 % p/p ;
(4) un alcool sélectionné parmi l'éthanol, le THFA, et les mélanges de ceux-ci, à une concentration de 15 à 25 % p/p ;
(5) un composant sélectionné parmi les esters glycéryliques de polyoxyéthylène, les PEG, et les mélanges de ceux-ci, à une concentration de 22 à 32 % p/p ; et
(6) un ester méthylique de glucose, à une concentration de 2 à 8 % p/p.

6. Formulation selon l'une quelconque des revendications précédentes, dans laquelle la concentration de l'acide salicylique dans la formulation est de 0,5 à 1,5 % p/p.

7. Formulation selon l'une quelconque des revendications précédentes, qui contient un ou plusieurs agents épaississants.

8. Formulation selon l'une quelconque des revendications précédentes, qui se trouve sous la forme d'une solution, d'une lotion, d'une crème ou d'un gel.

9. Formulation selon l'une quelconque des revendications précédentes, qui comprend en outre de l'acide usnique ou un usnate.

10. Produit qui incorpore une formulation selon l'une quelconque des revendications précédentes.

11. Formulation selon l'une quelconque des revendications 1 à 9, pour son utilisation dans un traitement réalisé sur un corps humain ou un animal vivant, en particulier humain.

12. Formulation selon l'une quelconque des revendications 1 à 9, pour son utilisation selon la revendication 11, dans laquelle l'affection est l'acné.

13. Formulation selon l'une quelconque des revendications 1 à 9, pour son utilisation dans le traitement d'une affection (notamment une affection de la peau ou de la structure de la peau) qui est susceptible d'être traitée par un agent kératolytique, ou d'une affection qui est provoquée, transmise et/ou exacerbée par une activité microbienne.

14. Formulation selon l'une quelconque des revendications 1 à 9, et/ou système solvant selon l'une quelconque des revendications 1 à 9, pour son utilisation dans l'amélioration de l'administration d'acide salicylique à un site cible dans ou sur la peau.
